# EUROPEAN PATENT APPLICATION

(11) **EP 1 666 485 A1**
(43) Date of publication of application: **07.06.2006**
(21) Application number: 04773605.3
(22) Date of filing: 14.09.2004
(51) Int. Cl.: C07H 19/044, A61K 31/7056, A61P 35/00

(54) **NOVEL INDOLOPYRROLOCARBAZOLE DERIVATIVE WITH ANTITUMOR ACTIVITY**

(30) Priority: 16.09.2003 JP 2003322550
(71) Applicant: BANYU PHARMACEUTICAL CO., LTD., Chuo-ku, Tokyo 103-8416 (JP)
(72) Inventor: YAMADA, Koji, c/o BANYU PHARMACEUTICAL CO. LTD., Tsukuba-shi, Ibaraki 3002611 (JP); SUNAMI, Satoshi, c/o BANYU PHARMACEUTICAL CO. LTD., Tsukuba-shi, Ibaraki 3002611 (JP); HIROSE, Masaaki, c/o BANYU PHARMACEUTICAL CO. LTD., Chuo-ku, Tokyo 1030026 (JP); OHKUBO, Mitsuru, c/o BANYU PHARMACEUTICAL CO., LTD, Tsukuba-shi,Ibaraki 3002611 (JP); ARAKAWA, Hiroharu, c/o BANYU PHARMACEUTICAL CO LTD, Tsukuba-shi , Ibaraki 3002611 (JP)
(74) Representative: Rollins, Anthony John
(86) International application number: PCT/JP2004/014661
(87) International publication number: WO 2005/026185

(57) **Abstract**

The present invention relates to a novel indolopyrrolocarbazole derivative which is represented by the formula [I]: 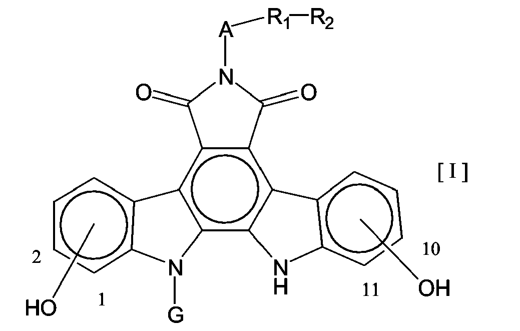 wherein:
A represents O, NH, or CH₂;
R₁ represents a single bond, a lower alkyl group, a lower alkenyl group, a lower alkynyl group, etc.;
R₂ represents a phenyl group, a naphthyl group, or a five- or six-membered aromatic or aliphatic heterocyclic ring having at least one atom selected from N, S, or O, wherein the phenyl group, naphthyl group, aromatic or aliphatic heterocyclic ring may be substituted; and
G represents a hexose group or a pentose group, or a pharmaceutically acceptable salt thereof

## Description

### Technical Field

The present invention relates to a novel indolopyrrolocarbazole derivative which is useful in the pharmaceutical field and, to be specific, inhibits the growth of tumor cells and exhibits an antitumor effect and also to a use thereof.

### Background Art

In the field of cancer chemotherapy, many compounds have been already practically used as pharmaceuticals. However, their effects are not always sufficient for various kinds of tumors and, in addition, the problem of resistance of tumor cells to such pharmaceuticals also makes their clinical use complicated.

Under such circumstances, development of novel anticancer substances is always demanded in the field of cancer therapy. Particularly, there has been a necessity for substances which overcome the resistance to the already-available anticancer substances and exhibit effectiveness to the types of cancer for which the already-available anticancer substances do not fully achieve the effect.

In view of such a present state, we have found novel indolopyrrolocarbazole derivatives having an anticancer activity and filed patent applications for a series of the compounds (specification of the U.S. Patent No. 5,591,842; specification of the U.S. Patent No. 5,668,271; specification of the U.S. Patent No. 5,804,564; specification of the U.S. Patent No. 5,922,860; pamphlet of the International Publication No. 95/30682; pamphlet of the International Publication No. 96/04293; Unexamined Japanese Patent Publication No. 10/245,390; etc.).

### Disclosure of the Invention

It is the problem which is to be solved by the present invention that chemical modification is applied to the indolopyrrolocarbazole-type antitumor substances disclosed in the above-mentioned patent applications so as to find the compounds having an excellent antitumor activity.

The present inventors have synthesized broad varieties of indolopyrrolocarbazole derivatives and reviewed their antitumor activity and, as a result, they have found that the compounds of the following formula [I] exhibit an excellent antitumor action to complete the present invention.

Thus, the present invention relates to a compound of the following formula or a pharmaceutically acceptable salt thereof: 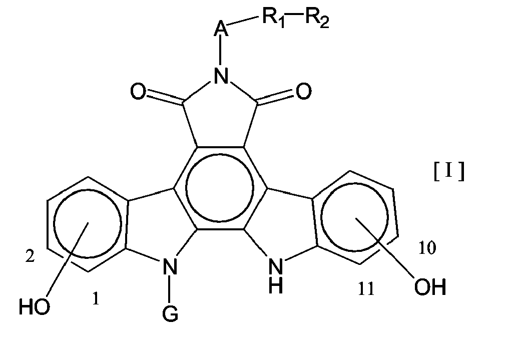 wherein:
A represents O, NH, or CH₂;
R₁ represents a single bond, a lower alkyl group, a lower alkenyl group, a lower alkynyl group, or a formula of Y₁- W, wherein Y₁ represents a lower alkyl group, a lower alkenyl group or dioxanyl; W represents a single bond or an oxygen atom; said lower alkyl group, lower alkenyl group, or lower alkynyl group may be substituted with one or more substituent(s), which is/are same or different, selected from the group consisting of <substituent group β>;
R₂ represents a phenyl group, a naphthyl group, or a five- or six-membered aromatic or aliphatic heterocyclic ring having at least one atom selected from N, S, or O, selected from the group consisting of <substituent group α>, wherein said phenyl group, naphthyl group, aromatic or aliphatic heterocyclic ring may be substituted with one or more substituent(s), which is/are the same or different, selected from the group consisting of <substituent group β> and/or a lower alkyl group substituted with one or more substituent(s), which is/are the same or different, selected from the group of <substituent group β>; when A represents an oxygen atom, R₂ may represent hydrogen atom; with a proviso that A is NH and R₁ is CH₂, R₂ is not any of a substituted phenyl group, a hydroxymethyl-substituted naphthyl group, an unsubstituted pyridyl group, a hydroxymethyl-substituted pyridyl group, an unsubstituted thienyl group, a hydroxymethyl-substituted 2-thienyl group, a mono(hydroxymethyl)-substituted 3-thienyl group, an unsubstituted furyl group and a hydroxymethyl-substituted furyl group;
G represents a hexose group or a pentose group;
<substituent group α> represents the following: 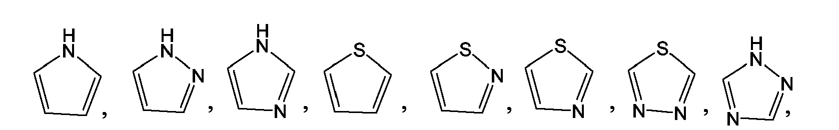 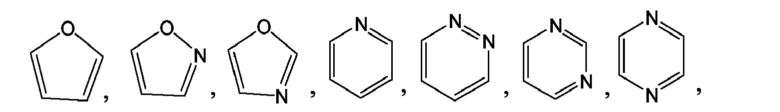 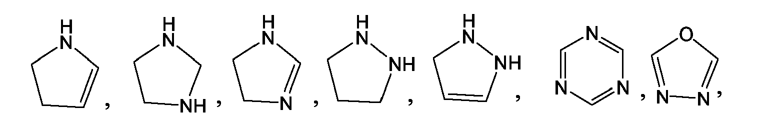 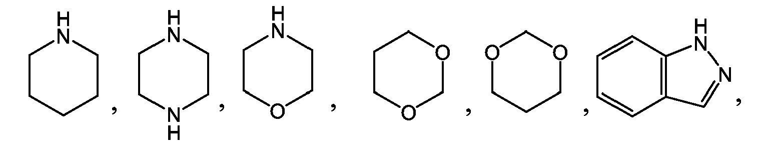 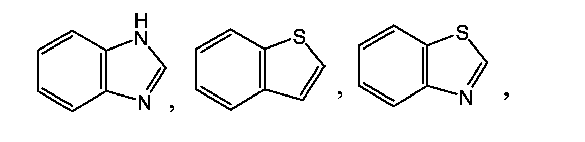 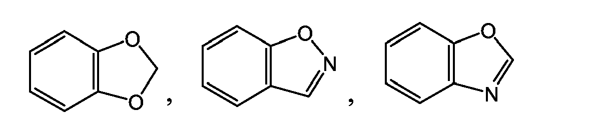 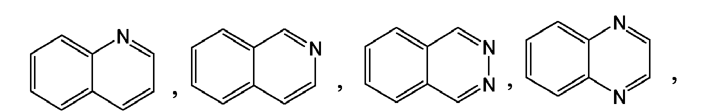 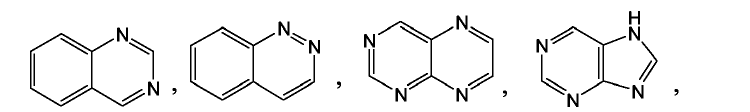 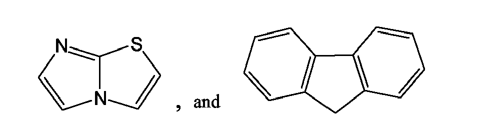 and; <substituent group β> represents the following:
a hydroxyl group, a cyano group, a halogen atom, a nitro group, a carboxyl group, a carbamoyl group, a formyl group, a lower alkanoyl group, a lower alkanoyloxy group, a lower alkoxy group, a hydroxyl lower alkoxy group, a lower alkoxy carbonyl group, a lower alkyl carbamoyl group, a di-lower alkyl carbamoyl group, a carbamoyloxy group, a lower alkyl carbamoyloxy group, a di-lower alkyl carbamoyloxy group, an amino group, a lower alkyl amino group, a di-lower alkyl amino group, a tri-lower alkyl ammonio group, a lower alkanoyl amino group, an aroyl amino group, a lower alkanoyl amidino group, a hydroxyl imino group, a lower alkoxy imino group, a lower alkyl thio group, a lower alkyl sulfinyl group, a lower alkyl sulfonyl group, a lower alkyl sulfonylamino group and a sulfamoyl group.

### Best Mode for Carrying Out the Invention

The present invention relates preferably to the compound represented by the formula [I] or the pharmaceutically acceptable salt thereof wherein G represents β-glucopyranosyl group; the positions of substitution of the OH groups on the indolopyrrolocarbazole ring are the 2- and 10-positions; R₁ represents a lower alkyl group; and R₂ represents a five- or six-membered aromatic or aliphatic heterocyclic ring having at least one atom selected from N, S, or O, selected from <substituent group α>.

Further, the present invention relates preferably to the compound represented by the formula [I] or the pharmaceutically acceptable salt thereof wherein the <substituent group α> represents the following: 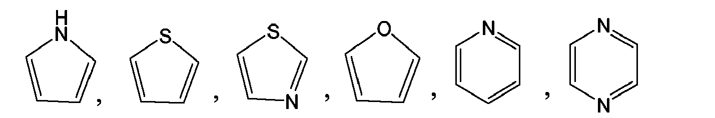 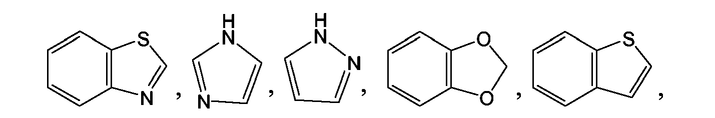 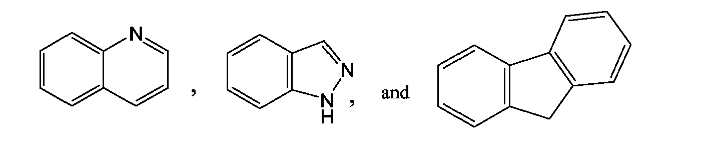 and the <substituent group β> represents the following:
a hydroxyl group, a nitro group, a lower alkanoyl group, a lower alkanoyloxy group, a lower alkoxy group, and a lower alkoxy carbonyl group.

Furthermore, the present invention relates more preferably to the compound represented by the formula [I] or the pharmaceutically acceptable salt thereof, wherein A is O; and the <substituent group α> represents the following: 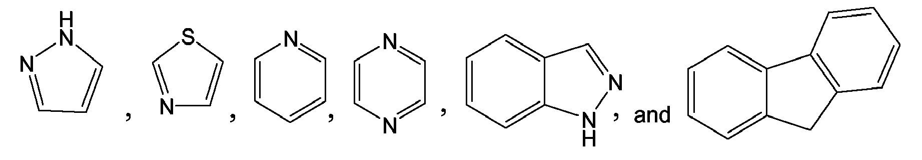 ; and the <substituent group β> represents the following:
a hydroxyl group, a lower alkanoyl group, a lower alkanoyloxy group, and a lower alkoxy carbonyl group;
   or, alternatively, relates more preferably to the compound represented by the formula [I] or the pharmaceutically acceptable salt thereof, wherein A is NH or CH₂; the <substituent group α> represents the following: 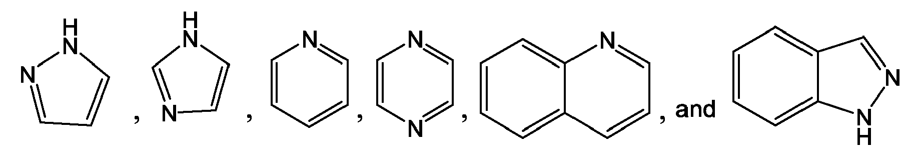 ; and the <substituent group β> represents the following:

a hydroxyl group, a lower alkanoyl group, and a lower alkoxy carbonyl group.

Also, the present invention relates particularly preferably to a compound which is:
6-N-(2,5-dihydroxymethyl-3-thienylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-pyrazinylmethylamino-12,13-dihydro-2,10-dihydroxy-12-β-D-glucopyranosyl-5H-indolo-[2,3-a]pyrrolo[3,4-c]carbazole-5,7(6H)-dione;
6-N-(4-quinolinylmethylamino)-12,13 -dihydro-2,10-dihydroxy-12-β-D-glucopyranosyl-5H-indolo[2,3-a]pyrrolo[3,4-c]carbazole-5,7(6H)-dione;
6-N-(4-nitro-2-1H-pyrrolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(2-1H-pyrrolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(3-1H-pyrazolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(4-1H-imidazolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(2-methoxycarbonyl-6-pyridylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(4-pyridylmethoxy)-12,13-dihydro-2,10-dihydroxy-12-β-D-glucopyranosyl-5H-indolo-[2,3-a]pyrrolo[3,4-c]carbazole-5,7(6H)-dione;
6-N-(6-hydroxymethyl-2-pyridylmethoxy)-12,13-dihydro-2,10-dihydroxy-12-β-D-glucopyranosyl-5H-indolo[2,3-a]pyrrolo[3,4-c]carbazole-5,7(6H)-dione;
6-N-(3-hydroxymethyl-4-pyridylmethoxy)-12,13-dihydro-2,10-dihydroxy-12-β-D-glucopyranosyl-5H-indolo[2,3-a]pyrrolo[3,4-c]carbazole-5,7(6H)-dione; or
6-N-(2-(4-pyridyl)ethyl)-12,13-dihydro-2,10-dihydroxy-12-β-D-glucopyranosyl-5H-indolo-[2,3-a]pyrrolo[3,4-c]carbazole-5,7(6H)-dione,
or a pharmaceutically acceptable salt thereof.

Still further, the present invention relates to a pharmaceutical composition comprising the compound of the formula (I) as an active ingredient, together with a pharmaceutically acceptable carrier or diluent; or to an antitumor agent comprising the compound of the formula (I) as an active ingredient, together with a pharmaceutically acceptable carrier or diluent.

Now the symbols and the terms used in the present specification will be illustrated.

The term "lower alkyl group" in the above formula (I) is an alkyl group of straight or branched chain having 1 to 6 carbons and its examples are methyl group, ethyl group, propyl group, isopropyl group, butyl group, isobutyl group, sec-butyl group, tert-butyl group, pentyl group and hexyl group. Among them, preferred examples are methyl group, ethyl group, propyl group, isopropyl group, tert-butyl group and pentyl group; and particularly preferred examples are methyl group, ethyl group, propyl group and isopropyl group.

The term "lower alkenyl group" in the above formula (I) is an alkenyl group of straight or branched chain having 2 to 6 carbons and its examples are vinyl group, 1-propenyl group, allyl group, isopropenyl group, 1-butenyl group, 3-butenyl group, 1,3-butanedienyl group, 2-pentenyl group, 4-pentenyl group, 1-hexenyl group, 3-hexenyl group and 5-hexenyl group. Among them, preferred examples are 1-propenyl group, allyl group, isopropenyl group and 1-butenyl group.

The term "lower alkynyl group" in the above formula (I) is an alkynyl group of straight or branched chain having 2 to 6 carbons and its examples are 2-propynyl group, 2-butynyl group, 3-butynyl group and 2-pentynyl group. Among them, preferred examples are 2-propynyl group and 2-butynyl group.

The term "pentose group" in the above formula (I) is, for example, ribose, arabinose, xylose or 2-deoxyribose; preferably, ribose or xylose; and, particularly preferably, ribose. Hydroxyl group(s) of the pentose group may be substituted with 1 to 3 substituents, which are the same or different, selected from the group consisting of hydrogen atom, a lower alkyl group, a lower alkylcarbonyloxy group, a lower alkoxy group and an amino group, or may be oxidized.

The term "hexose group" in the above formula (I) is, for example, allose, glucose, mannose, galactose, glucosamine, galactosamine, 2-deoxyglucose, 4-O-methylglucose, rhamnose or glucuronic acid; preferably, glucose, mannose, galactose or allose; more preferably, glocuse; and, particularly preferably, a β-glucopyranosyl group. Hydroxyl group(s) of the hexose group may be substituted with 1 to 3 same or different substituents selected from the group consisting of hydrogen atom, a lower alkyl group, a lower alkylcarbonyloxy group, a lower alkoxy group and an amino group, or may be oxidized.

With regard to the term "halogen atom" in the above formula (I), examples thereof include fluorine atom, chlorine atom, bromine atom and iodine atom. Among them, fluorine atom, chlorine atom and bromine atom are preferred and fluorine atom is more preferred.

The term "lower alkanoyl group" in the above formula (I) is a group where the above "lower alkyl group" is bonded to a carbonyl group, and a group where an alkyl group having 1 to 5 carbon(s) is bonded to a carbonyl group is preferred. Its examples are acetyl group, propionyl group, butyryl group, isobutyryl group, valeryl group, isovaleryl group, pivaloyl group and pentanoyl group and, among them, preferred examples are acetyl group, propionyl group and pivaloyl group.

The term "lower alkanoyloxy group" in the above formula (I) is a group where the term "lower alkanoyl group" is bonded to an oxygen atom. To be specific, examples thereof include acetyloxy group and propyloxy group and, among them, acetyoloxy group is for example preferred.

The term "lower alkoxy group" in the above formula (I) is a group where the term "lower alkyl group" is bonded to an oxygen atom, and examples thereof include methoxy group, ethoxy group, propoxy group, isopropoxy group, butoxy group, isobutoxy group, sec-butoxy group, tert-butoxy group, pentyloxy group, neopentyloxy group, hexyloxy group and isohexyloxy group. Among them, methoxy group, ethoxy group, isopropyloxy group and tert-butoxy group are preferred and methoxy group is particularly preferred.

The term "hydroxy lower alkoxy group" in the above formula (I) is a "lower alkoxy group" to which one or more hydroxyl groups are bonded, and examples thereof include hydroxymethoxy group, hydroxyethoxy group and hydroxypropoxy group. Among them, preferred examples are hydroxyethoxy group and hydroxypropoxy group and particularly preferred one is hydroxyethoxy group.

The term "lower alkoxy carbonyl group" in the above formula (I) is a group where the above "lower alkoxy group" is bonded to a carbonyl group and, to be specific, examples thereof include methoxycarbonyl group, ethoxycarbonyl group, propoxycarbonyl group, isopropoxycarbonyl group, butoxycarbonyl group, isobutoxycarbonyl group, sec-butoxycarbonyl group, tert-butoxycarbonyl group, pentyloxycarbonyl group, neopentyloxycarbonyl group, hexyloxycarbonyl group and isohexyloxycarbonyl group. Among them, preferred ones are methoxycarbonyl group, ethoxycarbonyl group, isopropyloxycarbonyl group and tert-butoxycarbonyl group and, particularly preferred ones are methoxycarbonyl group and ethoxycarbonyl group.

The term "lower alkylcarbamoyl group" in the above formula (I) is a substituent where the above "lower alkyl group" is N-substituted to a carbamoyl group and examples thereof include N-methylcarbamoyl group, N-ethylcarbamoyl group, N-propylcarbamoyl group, N-isopropylcarbamoyl group, N-butylcarbamoyl group, N-isobutylcarbamoyl group, N-tert-butylcarbamoyl group, N-pentylcarbamoyl group and N-hexylcarbamoyl group. Among them, N-methylcarbamoyl group, N-ethylcarbamoyl group and N-butylcarbamoyl group are preferred.

The term "di-lower alkyl carbamoyl group" in the above formula (I) is a substituent where the above "lower alkyl group" is N,N-di-substituted to a carbamoyl group and examples thereof include N,N-dimethylcarbamoyl group, N,N-diethylcarbamoyl group, N,N-dipropylcarbamoyl group, N,N-diisopropylcarbamoyl group, N,N-dibutylcarbamoyl group, N,N-diisobutylcarbamoyl group, N,N-di-tert-butylcarbamoyl group, N,N-dipentylcarbamoyl group, N,N-dihexylcarbamoyl group, N-ethyl-N-methylcarbamoyl group and N-methyl-N-propylcarbamoyl group. Among them, preferred examples are N,N-dimethylcarbamoyl group, N,N-diethylcarbamoyl group, N,N-dibutylcarbamoyl group, N-ethyl-N-methylcarbamoyl group and N-methyl-N-propylcarbamoyl group.

The term "lower alkylcarbamoyloxy group" in the above formula (I) is a group where the above "lower alkylcarbamoyl group" is bonded to an oxygen atom, and examples thereof include N-methylcarbamoyloxy group, N-ethylcarbamoyloxy group, N-propylcarbamoyloxy group, N-isopropylcarbamoyloxy group, N-butylcarbamoyloxy group, N-isobutylcarbamoyloxy group, N-tert-butylcarbamoyloxy group, N-pentylcarbamoyloxy group and N-hexylcarbamoyloxy group. Among them, preferred examples are N-methylcarbamoyloxy group, N-ethylcarbamoyloxy group and N-butylcarbamoyloxy group.

The term "di-lower alkylcarbamoyloxy group" in the above formula (I) is a substituent where the above "di-lower alkylcarbamoyl group" is bonded to an oxygen atom and examples thereof include N,N-dimethylcarbamoyloxy group, N,N-diethylcarbamoyloxy group, N,N-dipropylcarbamoyloxy group, N,N-diisopropylcarbamoyloxy group, N,N-dibutylcarbamoyloxy group, N,N-diisobutylcarbamoyloxy group, N,N-di-tert-butylcarbamoyloxy group, N,N-dipentylcarbamoyloxy group, N,N-dihexylcarbamoyloxy group, N-ethyl-N-methylcarbamoyloxy group and N-methyl-N-propylcarbamoyloxy group. Among them, preferred examples are N,N-dimethylcarbamoyloxy group, N,N-diethylcarbamoyloxy group, N,N-dibutylcarbamoyloxy group, N-ethyl-N-methylcarbamoyloxy group and N-methyl-N-propylcarbamoyloxy group.

The term "lower alkylamino group" in the above formula (I) is a substituent where the above "lower alkyl group" is N-substituted to an amino group and examples thereof include N-methylamino group, N-ethylamino group, N-propylamino group, N-isopropylamino group, N-butylamino group, N-isobutylamino group, N-tert-butylamino group, N-pentylamino group and N-hexylamino group. Among them, preferred examples are N-methylamino group, N-ethylamino group and N-butylamino group.

The term "di-lower alkylamino group" in the above formula (I) is a substituent where the above "lower alkyl group" is N,N-di-substituted to an amino group and examples thereof include N,N-dimethylamino group, N,N-diethylamino group, N,N-dipropylamino group, N,N-diisopropylamino group, N,N-dibutylamino group, N,N-diisobutylamino group, N,N-di-tert-butylamino group, N,N-dipentylamino group, N,N-dihexylamino group, N-ethyl-N-methylamino group and N-methyl-N-propylamino group. Among them, preferred examples are N,N-dimethylamino group, N,N-diethylamino group, N,N-dibutylamino group, N-ethyl-N-methylamino group and N-methyl-N-propylamino group.

The term "tri-lower alkylammonio group" in the above formula (I) is a substituent where the above "lower alkyl group" is N,N,N-tri-substituted to an amino group and examples thereof include N,N,N-trimethylammonio group, N,N,N-triethylammonio group, N,N,N-tripropylammonio group, N,N,N-triisopropylammonio group, N,N,N-tributylammonio group, N,N,N-triisobutylammonio group, N,N,N-tri-tert-butylammonio group, N,N,N-tripentylammonio group, N,N,N-trihexylammonio group, N-ethyl-N,N-dimethylammonio group and N,N-dimethyl-N-propylammonio group. Among them, preferred examples are N,N,N-trimethylammonio group, N,N,N-triethylammonio group, N,N,N-tri utylammonio group, N-ethyl-N,N-dimethylammonio group and N,N-dimethyl-N-propylammonio group.

The term "lower alkanoylamino group" in the above formula (I) is a substituent where the above "lower alkanoyl group" is substituted to an amino group and examples thereof include N-acetylamino group, N-propionylamino group and N-butyrylamino group. Among them, preferred examples are N-acetylamino group and N-propionylamino group.

The term "aroylamino group" in the above formula (I) is a substituent where an aroyl group is substituted to an amino group and examples thereof include N-benzoylamino group and N-naphthylcarbonylamino group. Among them, preferred example is N-benzoylamino group.

The term "lower alkanoylamidino group" in the above formula (I) is a substituent where the above "lower alkanoyl group" is substituted to an amidino group and examples thereof include N-acetylamidino group, N-propionylamidino group and N-butyrylamidino group. Among them, preferred examples are N-acetylamidino group and N-propionylamidino group.

The term "lower alkoxyimino group" in the above formula (I) is a substituent where the above "lower alkoxy group" is substituted to an imino group and examples thereof include methoxyimino group, ethoxyimino group and propoxyimino group. Among them, preferred examples are methoxyimino group and ethoxyimino group.

The term "lower alkylthio group" in the above formula (I) is a group where the above "lower alkyl group" is bonded to sulfur atom and examples thereof include methylthio group, ethylthio group, propylthio group, isopropylthio group, butylthio group, isobutylthio group, tert-butylthio group, pentylthio group and hexylthio group. Among them, preferred examples are methylthio group, ethylthio group, butylthio group and tert-butylthio group.

The term "lower alkylsulfinyl group" in the above formula (I) is a group where the above "lower alkyl" is bonded to a sulfinyl group and examples thereof include methylsulfinyl group, ethylsulfinyl group and butylsulfinyl group. Among them, preferred examples are methylsulfinyl group and ethylsulfinyl group.

The term "lower alkylsulfonyl group" in the above formula (I) is a group where the above "lower alkyl" is bonded to a sulfonyl group and examples thereof include methylsulfonyl group, ethylsulfonyl group and butylsulfonyl group. Among them, preferred examples are methylsulfonyl group and ethylsulfonyl group.

The term "lower alkylsulfonylamino group" in the above formula (I) is a substituent where the above "lower alkylsulfonyl group" is N-substituted to an amino group and examples thereof include N-methylsulfonylamino group, N-ethylsulfonylamino group and N-butylsulfonylamino group. Among them, the preferred examples are N-methylsulfonylamino group and N-ethylsulfonylamino group.

Now the symbols in the above formula (I) will be illustrated.

A in the above formula (I) represents O, NH or CH₂ and, preferably, it is O or NH.

R₁ in the above formula (I) represents a single bond, a lower alkyl group, a lower alkenyl group, a lower alkynyl group, or a formula of Y₁-W, wherein Y₁ represents a lower alkyl group, a lower alkenyl group or dioxanyl; W represents a single bond or an oxygen atom; said lower alkyl group, lower alkenyl group, or lower alkynyl group may be substituted with one or more substituents, which are the same or different, selected from the <substituent group β>. Preferably, R₁ represents a lower alkyl group or a lower alkenyl group and, more preferably, it represents a lower alkyl group.

R₂ in the above formula (I) represents a phenyl group, a naphthyl group, or a five- or six-membered aromatic or aliphatic heterocyclic ring having at least one atom selected from N, S, or O, selected from the <substituent group α>, wherein said phenyl group, naphthyl group, aromatic or aliphatic heterocyclic ring may be substituted with one or more substituents, which are the same or different, selected from the <substituent group β> and/or a lower alkyl group substituted with one or more substituents, which are the same or different, selected from the <substituent group β>. When A represents an oxygen atom, R₂ may represent hydrogen atom. When A represents NH and R₁ represents CH₂, then R₂ is not any of a substituted phenyl group, a hydroxymethyl-substituted naphthyl group, an unsubstituted pyridyl group, a hydroxymethyl-substituted pyridyl group, an unsubstituted thienyl group, a hydroxymethyl-substituted 2-thienyl group, a mono(hydroxymethyl)-substituted 3-thienyl group, an unsubstituted furyl group and a hydroxymethyl-substituted furyl group. Preferably, R₂ represents a five- or six-membered aromatic or aliphatic heterocycle ring containing at least one N, S or O, selected from the <substituent group α>.

In the case where R₂ represents a five- or six-membered aromatic or aliphatic heterocycle ring containing at least one N, S or O, selected from the <substituent group α>, R₂ bonds to the adjacent R₁ (however, when R₁ is a single bond, R₂ bonds to the adjacent A) via an unitable atom (carbon atom or nitrogen atom) of the said aromatic or aliphatic heterocycle ring, which can bind thereto. Examples of the binding forms of five- or six-membered aromatic or aliphatic heterocycle ring containing at least one N, S or O, selected from the <substituent group α> include, but are not limited to, the following.
Examples of the binding forms of <substituent group α> are:

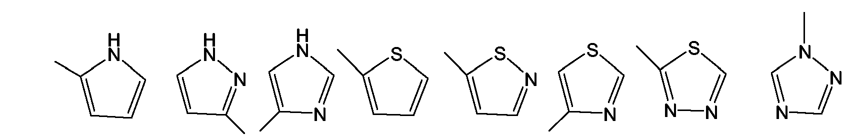

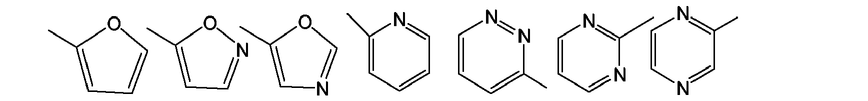

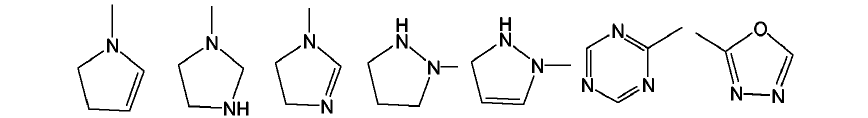

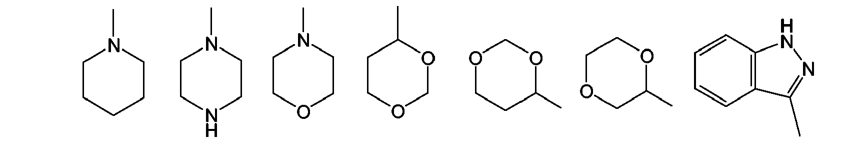

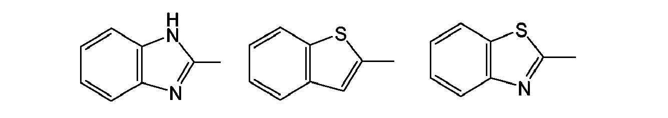

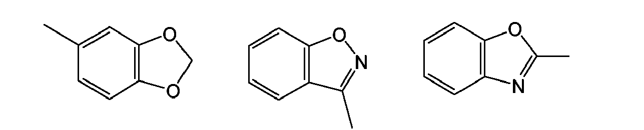

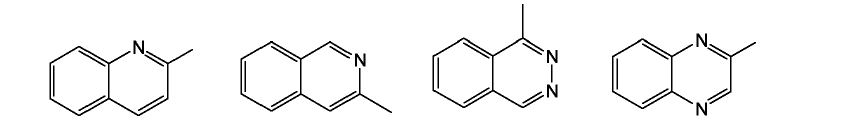

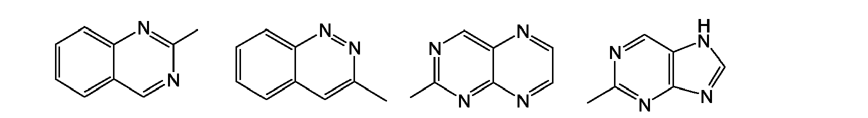

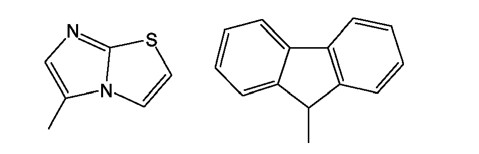

G in the above formula (I) represents a pentose group or a hexose group, preferably a hexose group and, more preferably, a β-glucopyranosyl group.

The positions of substitution of two hydroxyl groups on the indolopyrrolocarbazole skeleton in the above formula (I) are the 2- and 10-positions or the 1- and 11-positions and, preferably, the 2- and 10-positions.

The <substituent group α> in the above formula (I) is as follows:

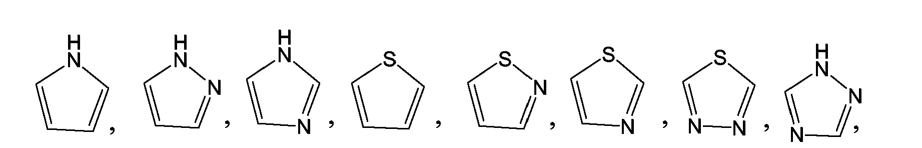

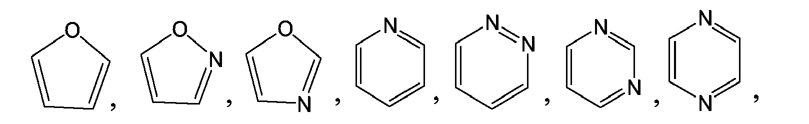

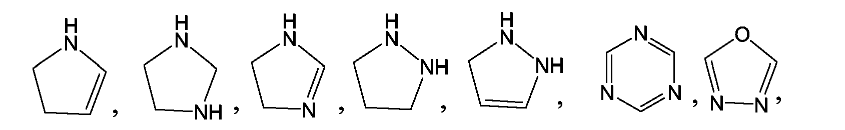

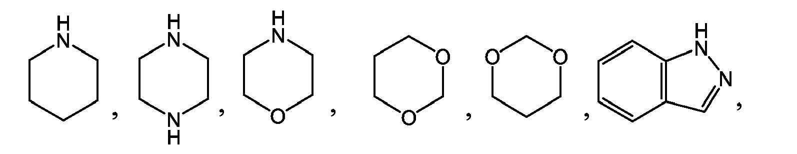

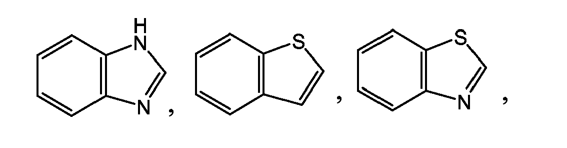

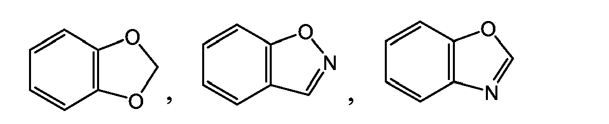

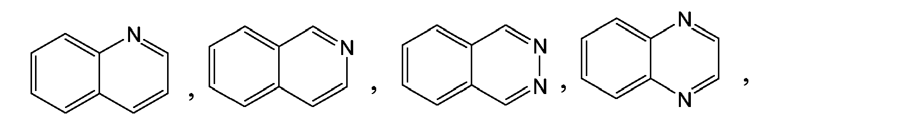

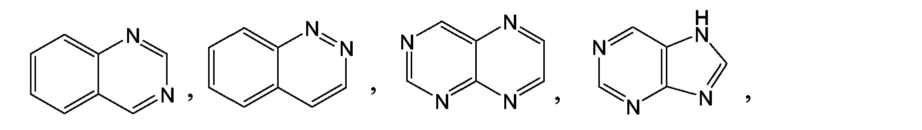

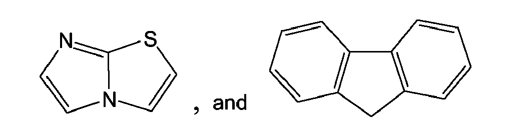

Preferably, the <substituent group *α>* in the above formula (I) is as follows:

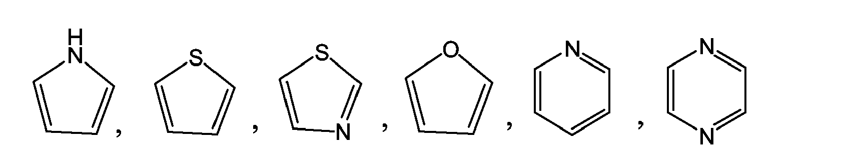

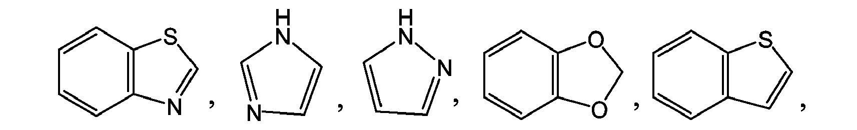

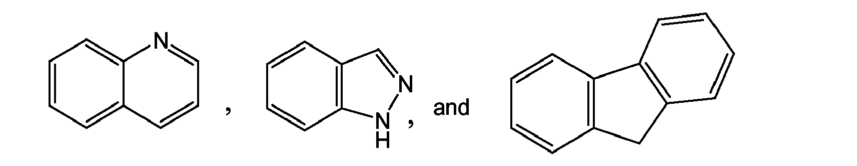

More preferably, the <substituent group *α>* in the above formula (I) is as follows: 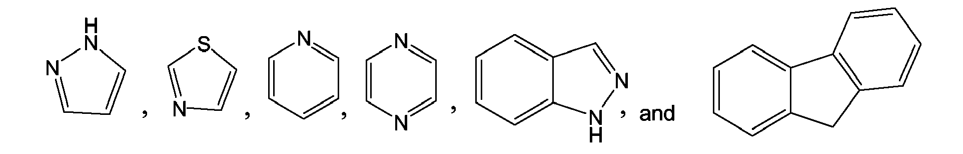 or 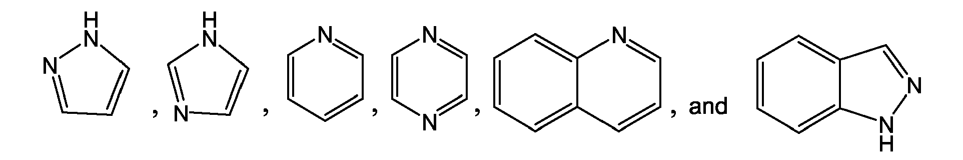 or 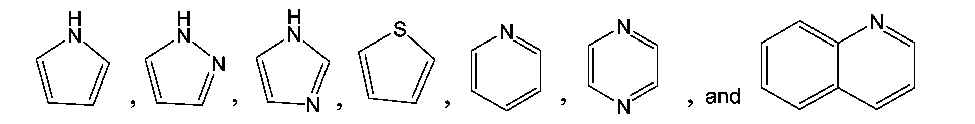 or

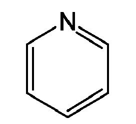

The <substituent group β> in the above formula (I) represents a hydroxyl group, a cyano group, a halogen atom, a nitro group, a carboxyl group, a carbamoyl group, a formyl group, a lower alkanoyl group, a lower alkanoyloxy group, a lower alkoxy group, a hydroxyl lower alkoxy group, a lower alkoxy carbonyl group, a lower alkyl carbamoyl group, a di-lower alkyl carbamoyl group, a carbamoyloxy group, a lower alkyl carbamoyloxy group, a di-lower alkyl carbamoyloxy group, an amino group, a lower alkyl amino group, a di-lower alkyl amino group, a tri-lower alkyl ammonio group, a lower alkanoyl amino group, an aroyl amino group, a lower alkanoyl amidino group, a hydroxyl imino group, a lower alkoxy imino group, a lower alkyl thio group, a lower alkyl sulfinyl group, a lower alkyl sulfonyl group, a lower alkyl sulfonylamino group and a sulfamoyl group;
preferably, it represents a hydroxyl group, a nitro group, a lower alkanoyl group, a lower alkanoyloxy group, a lower alkoxy group and a lower alkoxycarbonyl group;
more preferably, it represents a hydroxyl group, a nitro group, a lower alkanoyl group, a lower alkanoyloxy group, a lower alkoxy group, and a lower alkoxycarbonyl group; or represents a hydroxyl group, a lower alkanoyl group, a lower alkanoyloxy group and a lower alkoxycarbonyl group; or represents a hydroxyl group, a lower alkoxycarbonyl group, and a nitro group; and
particularly preferably, it represents a hydroxyl group.

Now, a process for the production of the compound of the present invention will be illustrated.

The indolopyrrolocarbazole derivative (the case where A = O, NH or CH₂ in the formula [I]) can be produced by the reaction of a compound of the formula [II] which is a publicly known compound mentioned in the European Patent Laid-Open Gazette 0528030 A1, the European Patent Laid-Open Gazette 0545195 A1, WO 95/30682 and WO 96/04293: 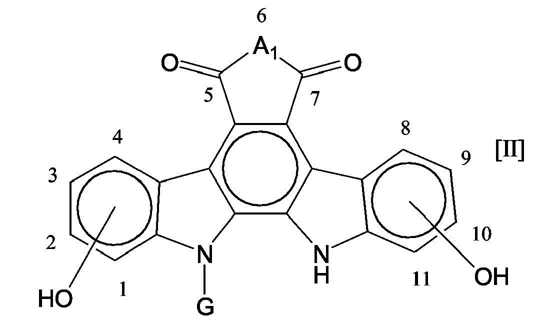 wherein A₁ represents NH or O; and G has the same meaning as mentioned above, with a compound of the formula [III]:

H₂N-Z-R₁-R₂ [III]

Wherein Z represents O, NH or CH₂; and R₁ and R₂ have the same meanings as mentioned above.

Here, reaction of the compound represented by the formula [II] with the compound of the formula [III] (in which Z represents NH) is a reaction of an imide or an acid anhydride with a hydrazine derivative, which has been widely known in the field of chemistry Also, the reaction of the compound represented by the formula [II] (in which A₁ represents O) with the compound of the formula [III] (in which Z represents O or CH₂) is a reaction of an acid anhydride with an amine derivative or a hydroxyamine derivative, which has been widely known in the field of chemistry. These reactions can be carried out using a solvent which usually does not have an unfavorable influence on the reaction, such as tetrahydrofuran, N,N-dimethylformamide or N-methylpyrrolidone; and the amount of the compound of the formula [III] used is usually from a bit excessive to five molar equivalents to that of the compound of the formula [II], although, if necessary, it is possible to use a greatly excessive amount thereof The reaction temperature is usually within a range of -50°C to the boiling of the solvent and, if necessary, the temperature which is higher or lower than the aforementioned one. The reaction time is usually within a range of 30 minutes to 2 days and, if necessary, longer or shorter time than that may be applied.

When A represents NH in the formula [I], the indolopyrrolocarbazole derivative of the present invention can be produced by subjecting to condensation a compound of the formula [IV]: 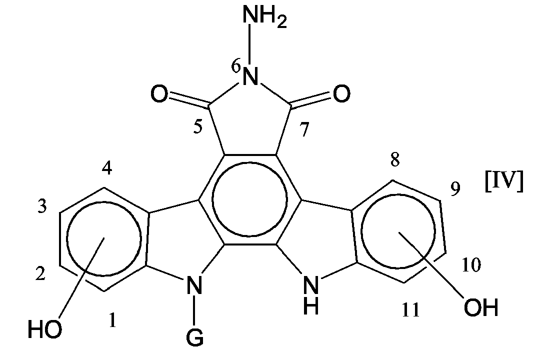 wherein G has the above-mentioned meaning,
and a compound of the formula [V]:

R₂-R₁-CHO [V]

wherein R₁ and R₂ have the above-mentioned meanings or the groups where hydroxyl groups existing in R₁ and R₂ are protected; then reducing the resulting compound; and, if necessary, removing protective groups thereof

That is, the compound of the formula [I] in which A is NH can be prepared by condensing the compound of the formula [IV] and the compound of the formula [V], and then the resulting compound is reduced to give the compound of the formula [I]. The reaction may be carried out in the same reaction system but, if necessary, a Schiff base (hydrazone) which is an intermediate product may be temporarily isolated. Usually, the compound of the formula [IV] and the compound of the formula [V] are mixed in an appropriate solvent and then a reducing agent is added thereto. At that time, it is preferred to conduct the reaction in the presence of an acid such as acetic acid or hydrochloric acid. The solvent used here include, for example, an alcoholic solvent such as methanol or ethanol and an aprotic polar solvent such as tetrahydrofuran or N,N-dimethylformamide. The reduction of the Schiff base may be carried out using a metal hydride complex such as sodium cyanoborohydride, or may be carried out by a catalytic reduction using palladium or the like.

Further, when A represents NH in the formula [I], the indolopyrrolocarbazole derivative of the present invention can be produced by the reaction of the compound of the formula [IV] with a compound of the formula [VI]:

R₂-R₁-L [VI]

wherein L is a leaving group; and R¹ and R² have the same meanings as mentioned above, followed, if necessary, by removing the protective group.

Thus, the compound of the formula [I] in which A represents NH can be produced by an alkylation reaction of an amine comprising the reaction of a compound of the formula [IV] with a compound of the formula [VI]; for example, it may be carried out by a known method such as the reaction with an alkyl halide, an alkyl mesylate or an alkyl tosylate.

The product in the above reaction may be purified by known methods in the field of organic synthetic chemistry such as a precipitation method, a solvent extraction method, recrystallization and chromatography

The compound of the formula [III], [V] or [VI] is a publicly known compound or can be easily produced from a known compound by a method which is publicly known or commonly used by persons skilled in the art. Also, the compound of the formula [IV] can be produced according to the Examples in the specification of the Japanese Patent No. 2,629,542 or by a similar process thereto.

The present invention also relates to a pharmaceutically acceptable salt of the compound as prepared by the above process. With regard to such a salt, examples thereof include a salt with an alkaline metal such as potassium and sodium; a salt with an alkaline earth metal such as calcium; a salt with a basic organic compound such as ethylamine and arginine; a salt with an inorganic acid such as hydrochloric acid and sulfuric acid; and a salt with an organic acid such as acetic acid, citric acid and maleic acid.

A process for the preparation of a pharmaceutically acceptable salt of the compound according to the present invention may be carried out by an appropriate combination of processes, which are usually used in the field of organic synthetic chemistry. Specifically there is mentioned a process where a solution of the compound of the present invention in a free form is subjected to neutralization and titration with an alkaline or acidic solution.

As shown in the following Table 1 and Table 2, the compound of the formula [I] of the present invention shows an excellent cell-growth suppressive effect against cancer cells (MKN-45 or HCT 116) derived from humans.

### Method for Judging the Pharmaceutical Effect using Cells

a) Reagents
   Fetal calf serum (FCS) was purchased from Molgate and a DMEM medium was purchased from Asahi Techno Glass.
b) Cells
   MKN-45, human stomach cancer cell, was purchased from the Immune Biology Laboratory. HCT 116, human colorectal cancer cell, was purchased from the American Type Culture Collection (ATCC).
c) Method for judging the effect
   Cells were suspended in a DMEM medium to which 10% of FCS were added, and a cell suspension was dispensed to a 96-well plastic plate to an extent of 1000 cells/50 microliters per well. Incubation was carried out at 37°C for one night in 5% CO₂-95% air. Each medicament was subjected to a gradient dilution using dimethyl sulfoxide or an appropriate solvent and each 50 microliters were dispensed to a microplate to which cells were sowed previously. Incubation was carried out for 3 days more at 37°C in 5% CO₂-95% air. Growth of the cells after incubation was measured by a WST-8 method (H. Tominaga, et al., *Anal. Commun.,* 36, 47-50 (1999). Here, the WST-8 method is a method in which 10 microliters of a WST-8 reagent solution are added to each well; incubation is continued at 37°C for 1 to 6 hours; the plate is stirred and the amount of formazan produced thereby is measured by a colorimetric method whereby the inhibiting rate of the medicament is determined. Thus, a 50% growth inhibition concentration (IC₅₀) was determined.

**[Table 1] Cell-Growth Suppressive Effect of Medicament against MKN-45 (Human Stomach Cancer Cells)**

| Example Nos. | 50% Growth Inhibiting Activity (IC₅₀, µM) |
|---|---|
| | MKN-45 Cells |
| 4 | 0.0012 |
| 5 | 0.0028 |
| 6 | 0.0014 |
| 10 | 0.004 |
| 12 | 0.002 |

**[Table 2] Cell-Growth Suppressive Effect of Medicament against HCT-116 (Human Colorectal Cancer Cells)**

| Example Nos. | 50% Growth Inhibiting Activity (IC₅₀, µM) |
|---|---|
| | HCT-116 Cells |
| 14 | 0.0012 |
| 15 | 0.0016 |
| 16 | 0.0049 |
| 18 | 0.0023 |
| 19 | 0.0037 |
| 30 | 0.00076 |
| 39 | 0.0050 |

As will be apparent from the results of the above pharmacological tests, the compound of the present invention exhibits an excellent cell-growth suppressive effect against cancer cells and is therefore useful for prevention and treatment of the diseases as an antitumor agent, particularly for treatment of cancer. Thus, a pharmaceutical composition containing the novel indolopyrrolocarbazole derivative according to the present invention, or a pharmaceutically acceptable salt thereof; or an anticancer agent containing the novel indolopyrrolocarbazole derivative according to the present invention, or a pharmaceutically acceptable salt thereof, is believed to be effective in the treatment of patients suffering from cancer. The pharmaceutical composition and the anticancer agent may contain a pharmaceutically acceptable carrier or diluent. Here, The term "pharmaceutically acceptable carrier or diluent" means excipients [such as fat, beeswax, semisolid and liquid polyol and natural or hydrogenated oil]; water (such as distilled water, particularly distilled water for injection), physiological saline solution, alcohol (such as ethanol), glycerol, polyol, aqueous solution of glucose, mannitol, plant oil, etc.; additives [such as filler, disintegrating agent, binder, lubricant, moisturizer, stabilizer, emulsifier, dispersing agent, preservative, sweetener, coloring agent, condiment or aromatizing agent, concentrating agent, diluent, buffer substance, solvent or solubilizing agent, chemical for achieving a preserving effect, salt for changing osmotic pressure, coating agent or antioxidant], etc.

With regard to appropriate tumor against which the compound according to the present invention is expected to exhibit a therapeutic effect, examples thereof include human solid cancer. Examples of the human solid cancer are brain cancer, brain and neck cancer, esophageal cancer, thyroid cancer, small-cell cancer, non-small-cell cancer, breast cancer, stomach cancer, cystic and biliary cancer, hepatic cancer, pancreatic cancer, colonic cancer, rectal cancer, ovarian cancer, chorioepithelioma, uterine body cancer, uterine neck cancer, pyeloureteric cancer, bladder cancer, prostatic cancer, penile cancer, testicular cancer, embryonal cancer, Wilms' cancer, skin cancer, malignant melanoma, neuroblastoma, osteosarcoma, Ewing's sarcoma and soft tissue sarcoma.

With regard to the dosage form when the compound of the present invention is used as an antitumor agent, it is possible to select various forms and the examples are oral preparations such as tablets, capsules, diluted powder, granules and liquid and sterilized liquid parenteral preparations such as solution and suspension.

Solid preparations may be manufactured in a form of tablets, capsules, granules or powder using the compound of the invention as it is, or may be manufactured using an appropriate additive. With regard to such additive, examples thereof include commonly used additives including saccharides such as lactose or glucose; starches such as corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; fatty acid salts such as calcium stearate or magnesium stearate; alcohols such as stearyl alcohol or benzyl alcohol; synthetic cellulose derivatives such as methylcellulose, carboxymethylcellulose, ethylcellulose or hydroxypropyl methylcelluose; and others such as water, gelatin, talc, vegetable oil, acacia, etc.

These tablet, capsule, granule and powder usually contain 0.1 to 100% or, preferably, 5 to 100% by weight of an effective ingredient.

Liquid preparation is manufactured in a form of suspension, syrup, injection, etc. using an appropriate additive which is commonly used in liquid preparations such as water, alcohols, or oils derived from plant such as, for example, peanut oil or sesame oil.

Particularly, with regard to an appropriate solvent in the case of administration by means of intramuscular injection, intravenous injection or subcutaneous injection in a parenteral route, examples thereof include distilled water for injection, aqueous solution of lidocaine hydrochloride (for intramuscular injection), physiological saline solution, aqueous solution of glucose, ethanol, polyethylene glycol, liquid for intravenous injection (e.g., aqueous solution of citric acid or sodium citrate), solution of an electrolyte (for intravenous dripping and intravenous injection) or a mixed solution thereof.

The injection solution may be in the form of a solution in which the active ingredient is dissolved in advance, or in a form of powder per *se* or in a form where a product to which an appropriate additive is added is dissolved in use. The injection solution usually contains 0.1 to 10% by weight or, preferably, 1 to 5% by weight of an effective ingredient.

The liquid preparation such as suspension or syrup for oral administration contains 0.5 to 10% by weight of an effective ingredient.

It is to be noted that preferred dose of the compound of the present invention in actual use varies depending upon the type of the compound used, the type of the composition mixed, frequency of administration and specific site to be treated, and host and tumor to be treated. For example, the dose per day for an adult is 10 to 500 mg in the case of oral administration and 10 to 100 mg in the case of parenteral administration, preferably intravenous injection, per day. Frequency of the administration varies depending upon the administration method and the symptom, and is one to five times. It is also possible to use an administration method such as an intermittent administration where administration is conducted every other day, every three days, etc.

### Examples

The present invention will now be more specifically illustrated by way of the following Examples although the present invention is not limited to those Examples only.

Meanings of the abbreviations in the NMR measurement are as follows.
- s:: singlet
- d:: doublet
- dd:: double doublet
- t:: triplet
- dt:: double triplet
- q:: quartet
- m:: multiplet
- br:: broad
- J:: coupling constant
- Hz:: hertz
- DMSO-d6:: heavy dimethyl sulfoxide

Meanings of the abbreviations in the mass measurement are as follows.
- FAB (m/z):: Fast atom bombardment
- ESI (m/z):: Electrospray ionization
- APCI (m/z):: Atmospheric pressure chemical ionization

### Example 1

Synthesis of the compound represented by the following formula:

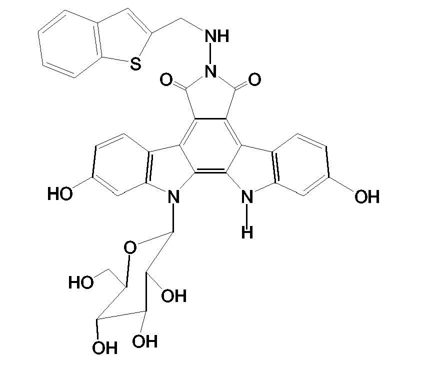

Process for the production of the compound A as shown below (hereinafter, referred to as "compound A") is disclosed in Example 47 of the specification of Japanese Patent No. 2,629,542 and Example 47 of the specification of US Patent No. 5591842.

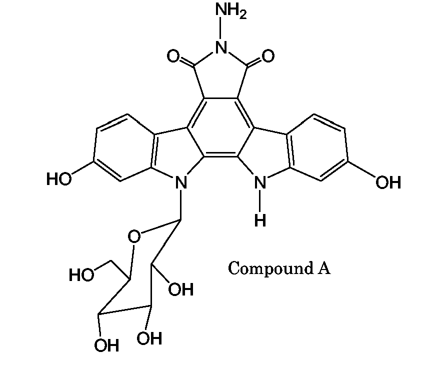

The compound A (53 mg) and 2-benzothiophene carboaldehyde (48 mg) were dissolved in methanol (MeOH) (4 ml), and a small amount of acetic acid was added thereto, followed by stirring at 80°C for 2 hours. After concentrating the reaction solution, the separated solid was washed with chloroform and dried. The resulting solid (55 mg) was suspended in tetrahydrofuran (THF) (3 ml) and sodium cyanoborohydride (NaBH₃CN) (34 mg) was added thereto, followed by stirring at room temperature. After that, a 10% HCl (hydrochloric acid)-MeOH (0.3 ml) was dropped thereinto, followed by stirring for 90 minutes. An appropriate amount of water was added thereto and the mixture was extracted with a mixed solvent of ethyl acetate and methyl ethyl ketone. After the organic phase was concentrated, the residue was filled on a Sephadex LH-20 column chromatography and developed with methanol. The desired fraction was concentrated and dried to give the compound represented by the title formula (15 mg) as a red solid.

Rf value: 0.47 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 654 (M)⁺
¹H-NMR (300 MHz, DMSO-d6, δ ppm): 3.42-3.53 (2H, m), 3.70-4.08 (4H, m), 4.58 (2H, d, J = 4.3 Hz), 4.86-4.93 (1H, br), 5.03-5.18 (1H, br), 5.29-5.39 (1H, br), 5.80-5.92 (1H, br), 5.95 (1H, d, J = 8.2 Hz), 6.34 (1H, t, J = 4.3 Hz), 6.72-6.83 (2H, m), 6.96 (1H, s), 7.15 (1H, s), 7.23-7.30 (2H, m), 7.39 (1H, s), 7.67-7.73 (1H, m), 7.85-7.92 (1H, m), 8.77 (1H, d, J = 9.0 Hz), 8.84 (1H, d, J = 8.5 Hz), 9.51-10.09 (2H, br), 11.17 (1H, br).

### Example 2

Synthesis of the compound represented by the following formula

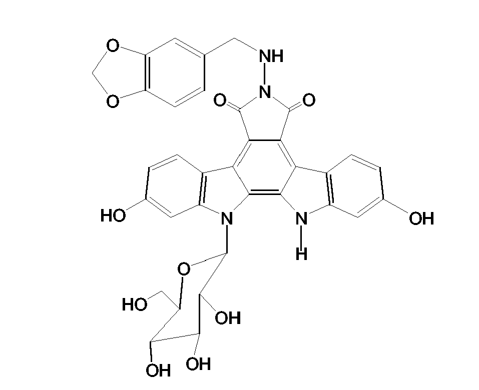

The compound A (50 mg) and piperonal (27 mg) were dissolved in methanol (10 ml) and then 30 ul of acetic acid was added thereto, followed by stirring at 80°C for 6 hours. After the reaction solution was concentrated, the residue was dissolved in methanol and solidified with chloroform-hexane. The resulting solid was dissolved in 12 ml of methanol-tetrahydrofuran (1:1), and NaBH₃CN (17 mg) and 10% HCl-MeOH (3 ml) were added thereto, followed by stirring for 90 minutes at room temperature. After the reaction solution was concentrated, the residue was filled on a Sephadex LH-20 column chromatography and developed with methanol. The desired fraction was concentrated and dried to give the desired compound (47.3 mg) as a red solid.

Rf value: 0.46 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 668 (M)⁺, 692 (M + Na)⁺
¹H-NMR (300 MHz, DMSO-d6, δ ppm): 3.50 (2H, br), 3.75-3.79 (1H, m), 3.90 (2H, br), 3.99-4.03 (1H, m), 4.17 (2H, d, J = 4.8 Hz), 4.91 (1H, d, J = 5.7 Hz), 5.11 (1H, d, J = 4.8 Hz), 5.33 (1H, d, J = 4.8 Hz), 5.86 (1H, t, J = 3.9 Hz), 5.94 (2H, s), 5.96 (1H, d, J = 10.5 Hz), 6.04 (1H, t, J = 5.1 Hz), 6.77-6.82 (3H, m), 6.86 (1H, dt, J = 1.2 Hz, 8.1 Hz), 6.97 (1H, d, J = 1.8 Hz), 7.11 (1H, d, J = 1.2 Hz), 7.17 (1H, d, J = 1.6 Hz), 8.78 (1H, d, J = 8.6 Hz), 8.86 (1H, d, J = 8.6 Hz), 9.74 (1H, s), 9.77 (1H, s), 11.18 (1H, s).

### Example 3

Synthesis of the compound represented by the following formula

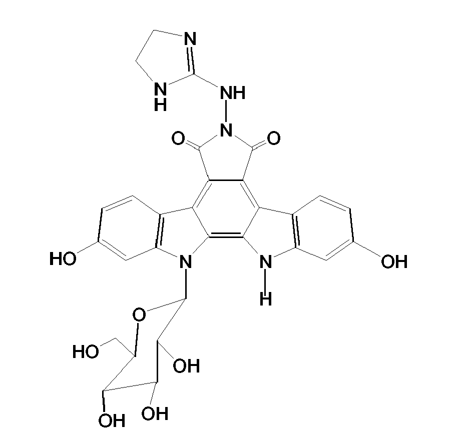

Process for the production of the compound B (hereinafter, referred to as "compound B") is disclosed in Example C of the specification of US Patent No. 5591842.

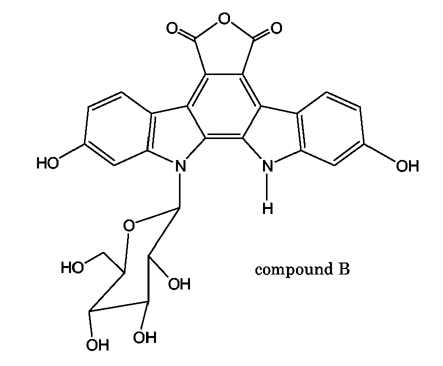

The compound B (110 mg), 2-hydrazino-2-imidazoline hydrobromide (115 mg) and triethylamine (80 µl) were dissolved in N,N-dimethylformamide (DMF) (5 ml), followed by stirring at 80°C for 6 hours. After concentrating the reaction solution, the residue was filled on a Sephadex LH-20 column chromatography and developed with methanol. The desired fraction was concentrated and dried to give the compound (67.1 mg) represented by the captioned formula as a red solid.
FAB (m/z): 603 (M + H)⁺
¹H-NMR (300 MHz, DMSO-d6, δ ppm): 11.31 (1H, s), 10.80 (1H, s), 9.86 (1H, s), 9.84 (1H, s), 8.81 (1H, d, J = 8.6 Hz), 8.74 (1H, d, J = 8.6 Hz), 7.21 (1H, d, J = 1.5 Hz), 7.01 (1H, d, J = 2.0 Hz), 6.81 (2H, m), 6.02 (1H, d, J = 8.6 Hz), 5.91 (1H, t, J = 3.4 Hz), 5.35 (1H, d, J = 1.8 Hz), 5.16 (1H, d, J = 5.3 Hz), 4.92 (1H, d, J = 4.9 Hz), 4.05 (2H, m), 3.92 (2H, br), 3.72 (4H, m), 3.51 (2H, m).

### Example 4

Synthesis of the compound represented by the following formula

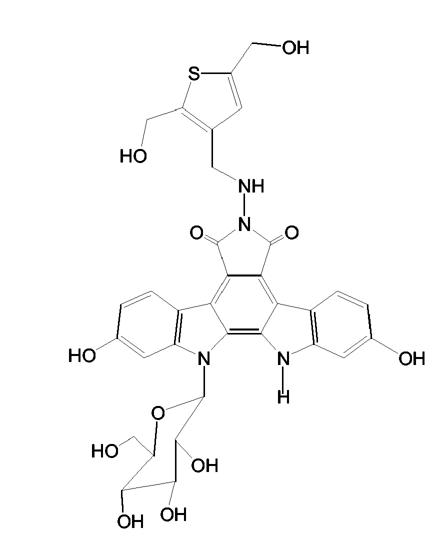

The compound A (50 mg) and 3-(2-tert-butyldimethylsilyloxymethyl-5-hydroxymethylthiophene) carboaldehyde (27 mg) were dissolved in methanol (10 ml) and then 50 µl of acetic acid was added thereto, followed by stirring at 80°C for 1 hour. After concentrating the reaction solution, the residue was dissolved in 10 ml of methanol-tetrahydrofuran (1:1) and then NaBH₃CN (40 mg) and 10% HCl-MeOH (3 ml) were added thereto, followed by stirring at room temperature for 60 minutes. After concentrating the reaction solution, the residue was filled on a Sephadex LH-20 column chromatography and developed with methanol. The desired fraction was concentrated and dried to give the compound represented by the captioned formula (15 mg).
FAB (m/z): 690 (M)⁺
¹H-NMR (300 MHz, DMSO-d6, δ ppm): 11.19 (1H, s), 9.78 (2H, br), 8.87 (1H, d, J = 8.4 Hz), 8.79 (1H, d, J = 9.0 Hz), 7.17 (1H, d, J = 1.8 Hz), 6.98 (1H, d, J = 1.8 Hz)k, 6.83 (1H, dd, J = 1. 8 Hz, 8.4 Hz), 6.81 (1H, dd, J = 1. 8 Hz, 8.1 Hz), 5.97 (1H, d, J = 8.1 Hz), 5.87 (1H, br), 5.82 (1H, t, J = 5.4 Hz), 5.34 (1H, t, J = 4.2 Hz), 5.3 (1H, d, J = 6.0 Hz), 5.29 (1H, t, J = 5.4 Hz), 5.12 (1H, br), 4.93 (1H, d, J = 4.2 Hz), 4.70 (2H, d, J = 5.1 Hz), 4.53 (2H, d, J = 4.8 Hz), 4.12 (2H, d, J = 6.6 Hz), 4.01 (1H, m), 3.91 (2H, s), 3.77 (1H, m), 3.51 (2H, m).

### Example 5

Synthesis of the compound represented by the following formula

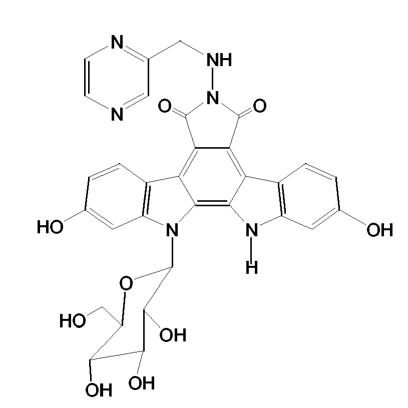

The compound A (50 mg) and 2-pyrazinecarboxyaldehyde (200 mg) were dissolved in methanol (10 ml) and then 100 µl of acetic acid was added thereto, followed by stirring at 80°C for 3 hours. After the reaction solution was neutralized with triethylamine, it was filled on a Sephadex LH-20 column chromatography and developed with methanol. The desired fraction was concentrated and dried to give a hydrazone compound (45 mg). The resulting hydrazone compound (20 mg) was dissolved in methanol-tetrahydrofuran (1:1) (10 ml) and then NaBH₃CN (20 mg) and 10% HCl-MeOH (3 ml) were added thereto, followed by stirring for 60 minutes. After the reaction solution was concentrated, a small amount of triethylamine was added to the residue and the mixture was filled on a Sephadex LH-20 column chromatography and developed with methanol. The desired fraction was concentrated and dried to give the compound represented by the above formula (6.0 mg) as a red solid.
FAB (m/z): 627 (M + H)⁺
¹H-NMR (300 MHz, DMSO-d6, δ ppm): 11.23 (1H, s), 9.03 (1H, d, J = 1.2 Hz), 8.82 (1H, d, J = 8.7 Hz), 8.74 (1H, d, J = 8.4 Hz), 8.51 (1H, d, J = 2.7 Hz), 8.46 (1H, dd, J = 1.2 Hz, 2.7 Hz), 7.19 (1H, d, J = 1.8 Hz), 7.03 (1H, d, J = 1.8 Hz), 6.83 (1H, dd, J = 1.8 Hz, 8.7 Hz), 6.80 (1H, dd, J = 1.8 Hz, 8.4 Hz), 6.43 (1H, t, J = 4.5 Hz), 6.18 (1H, br), 5.96 (1H, d, J = 8.4 Hz), 5.05 (1H, br), 4.43 (2H, d, J = 4.5 Hz), 4.05 (1H, m), 3.92 (2H, m), 3.77 (1H, m).

### Example 6

Synthesis of the compound represented by the following formula

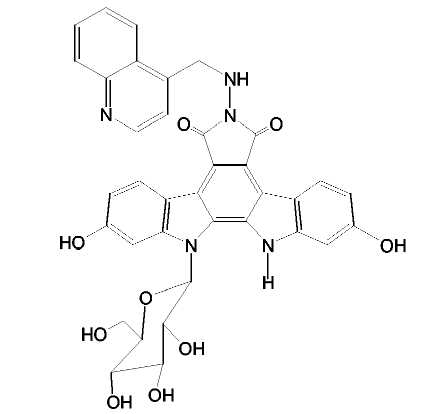

The compound A (50 mg) and 4-quinoline carboxyaldehyde (45 mg) were dissolved in methanol (10 ml); 100 µl of acetic acid was added thereto; and the mixture was stirred at 80°C through the night. The reaction solution was concentrated and the residue was solidified by addition of methanol-chloroform-n-hexane thereto. The resulting solid (10.2 mg) was dissolved in methanol-tetrahydrofuran (1:10) (5 ml), 5% palladium-carbon (10 mg) was added thereto and the mixture was stirred in a hydrogen atmosphere at room temperature for 60 minutes. The reaction solution was filtered through Celite to concentrate the filtrate. The residue was filled on a Sephadex LH-20 column chromatography and developed with methanol. The desired fraction was concentrated and dried to give the compound represented by the above formula (3.5 mg).

Rf value: 0.58 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 676 (M + H)⁺
¹H-NMR (300 MHz, DMSO-d6, δ ppm): 11.13 (1H, br), 8.86 (1H, d, J = 4.2 Hz), 8.85 (1H, d, J = 8.7 Hz), 8.76 (1H, d, J = 8.4 Hz), 8.50 (1H, d, J = 8.1 Hz), 8.03 (1H, d, J = 7.2 Hz), 7.79 (1H, d, J = 4.2 Hz), 7.76 (1H, dd, J = 2.1 Hz, 9.0 Hz), 7.69 (1H, dt, J = 1.5 Hz, 6.9 Hz), 7.12 (1H, s), 6.94 (1H, s), 6.78 (2H, 2H, dt, J = 1.8 Hz, 8.4 Hz), 6.34 (1H, t, J = 5.4 Hz), 5.94 (1H, d, J = 3.9 Hz), 4.95-5.40 (3H, br), 4.76 (2H, d, J = 5.4 Hz), 4.02 (1H, d, J = 10.5 Hz), 3.86-3.95 (2H, m), 3.77 (1H, d, J = 10.2 Hz), 3.50 (2H, m).

### Example 7

Synthesis of the compound represented by the following formula

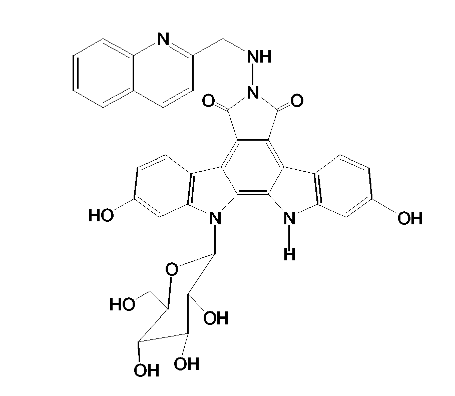

The compound A (50 mg) and 2-quinoline carboxyaldehyde (46.5 mg) were dissolved in methanol (10 ml); 100 µl of acetic acid was added thereto; and the mixture was stirred at 80°C for 1 hour. After the reaction solution was concentrated, the residue was filled on a Sephadex LH-20 column chromatography and developed with methanol. The yellow fraction was concentrated and dried to give 56.1 mg of the hydrazone compound. The resulting hydrazone compound(20 mg) was dissolved in tetrahydrofuran (5 ml), added to a solution of NaBH₃CN (110.5 mg) and 1M zinc dichloride diethyl ether solution (0.9 ml) in tetrahydrofuran (5 ml) and stirred through the night. To the reaction solution were added water and then a saturated aqueous solution of sodium hydrogen carbonate followed by extracting with ethyl acetate. The organic layer was washed with a saturated saline solution, then dried and concentrated. The residue was purified with a Diaion HP-20 column chromatography (methanol) and then with a Sephadex LH-20 column chromatography (methanol) to give the compound represented by the above formula (5.0 mg).

Rf value: 0.44 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 676 (M + H)⁺
¹H-NMR (300 MHz, DMSO-d6, δ ppm): 11.18 (1H, s), 9.78 (1H, s), 9.75 (1H, s), 8.83 (1H, d, J = 8.5 Hz), 8.75 (1H, d, J = 8.5 Hz), 8.39 (1H, d, J = 8.2 Hz), 7.99 (1H, d, J = 8.6 Hz), 7.93 (1H, d, J = 7.2 Hz), 7.87 (1H, d, J = 8.1 Hz), 7.63-7.68 (1H, m), 7.63 (1H, t, J = 7.8 Hz), 7.16 (1H, d, J = 2.1 Hz), 6.97 (1H, d, J = 1.8 Hz), 6.78-6.83 (2H, m), 6.43 (1H, t, J = 4.8 Hz), 5.95 (1H, d, J = 8.7 Hz), 5.86 (1H, t, J = 3.9 Hz), 5.33 (1H, d, J = 5.1 Hz), 5.10 (1H, d, J = 5.4 Hz), 4.88 (1H, d, J = 5.1 Hz), 4.56 (2H, d, J = 4.5 Hz), 3.99 (1H, m), 3.87 (1H, m), 3.49 (2H, m).

### Example 8

Synthesis of the compound represented by the following formula

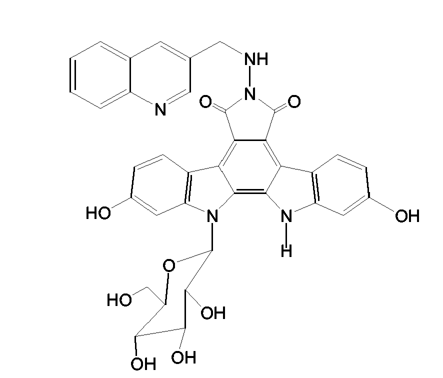

The compound A (50 mg) and 3-quinoline carboxyaldehyde (45 mg) were dissolved in methanol (10 ml); 100 µl of acetic acid was added thereto; and the mixture was stirred at 80°C for 6.5 hours. The reaction solution was concentrated and the residue was filled on a Sephadex LH-20 column chromatography and developed with methanol. The yellow fraction was concentrated and dried to give 56.7 mg of the hydrazone compound. The resulting hydrazone compound (20 mg) was dissolved in tetrahydrofuran (5 ml), and added to a tetrahydrofuran solution (5 ml) of NaBH₃CN (110.5 mg) and 1M zinc dichloride diethyl ether solution (0.9 ml) and stirred through the night. To the reaction solution were added water and then a saturated aqueous solution of sodium hydrogen carbonate, followed by extracting with ethyl acetate. The organic layer was washed with a saturated saline solution, then dried and concentrated. The residue was purified using a Sephadex LH-20 column chromatography (methanol) to give the compound represented by the above formula (3.0 mg).

Rf value: 0.48 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 676 (M + H)⁺
¹H-NMR (300 MHz, DMSO-d6, δ ppm): 11.16 (1H, s), 9.75 (2H, br), 9.08 (1H, d, J = 1.9 Hz), 8.82 (1H, d, J = 8.6 Hz), 8.74 (1H, d, J = 8.4 Hz), 8.37 (1H, br), 7.97 (1H, d, J = 7.8 Hz), 7.91 (1H, d, J = 7.8 Hz), 7.69 (1H, m), 7.54 (1H, t, J = 7.8 Hz), 7.15 (1H, d, J = 2.1 Hz), 6.96 (1H, d, J = 2.4 Hz), 6.80 (2H, m), 6.37(1H, t, J = 4.2 Hz), 5.94 (1H, d, J = 8.1 Hz), 5.86 (1H, br), 5.34 (1H, br), 5.11 (1H, br), 4.88 (1H, d, J = 4.8 Hz), 4.48 (2H, d, J = 4.5 Hz), 4.01 (1H, d, J = 10.5 Hz), 3.89 (2H, br), 3.76 (1H, d, J = 10.5 Hz), 3.50 (2H, m).

### Example 9

Synthesis of the compound represented by the following formula

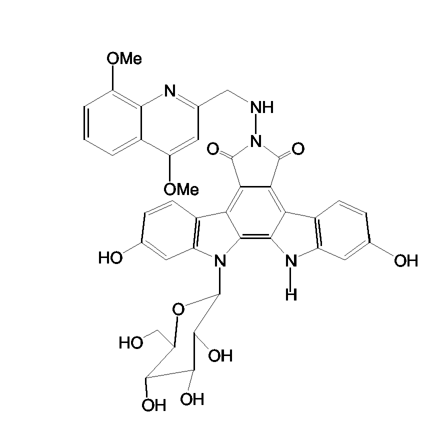

The compound A (10 mg) and 4,8-dimethoxy-2-quinoline carboxyaldehyde (8.3 mg) were dissolved in methanol (2 ml) and then 100 µl of acetic acid were added thereto, followed by stirring at 80°C for 4.5 hours. After the reaction solution was concentrated, the residue was filled on a Sephadex LH-20 column chromatography and developed with methanol and then with ethanol. A yellow fraction was concentrated and dried to give 4.0 mg of the hydrazone compound. The resulting hydrazone compound (4.0 mg) was dissolved in tetrahydrofuran (2 ml) and added to a solution of NaBH₃CN (0.3 mg) and 1M diethyl ether solution (0.17 ml) of zinc chloride in tetrahydrofuran (1 ml) were added thereto, followed by stirring through the night. To the reaction solution were added water and a saturated aqueous solution of sodium hydrogen carbonate, followed by extracting with ethyl acetate. The organic layer was washed with a saturated saline solution and dried/concentrated. The residue was purified using a Sephadex LH-20 column chromatography (methanol) to give the compound represented by the above formula (2.4 mg).
FAB (m/z): 736 (M + H)⁺
¹H-NMR (300 MHz, DMSO-d6, δ ppm): 11.19 (1H, s), 9.78 (2H, br), 8.85 (1H, d, J = 8.9 Hz), 8.78 (1H, d, J = 8.6 Hz), 7.68 (1H, s), 7.57 (1H, d, J = 8.4 Hz), 7.35 (1H, t, J = 8.1 Hz), 7.16 (1H, d, J = 1.6 Hz), 7.06 (1H, dd, J = 1.1 Hz, 7.2 Hz), 6.96 (1H, s), 6.78-6.84 (2H, m), 6.44 (1H, t, J = 3.5 Hz), 5.94 (1H, d, J = 7.8 Hz), 5.86 (1H, br), 5.32 (1H, br), 5.09 (1H, d, J = 4.3 Hz), 4.85 (1H, d, J = 4.5 Hz), 4.52 (2H, m), 4.15 (3H, s), 3.98 (1H, m), 3.89 (2H, m), 3.81 (3H, s), 3.75 (1H, m), 3.47 (2H, m).

### Example 10

Synthesis of the compound represented by the following formula

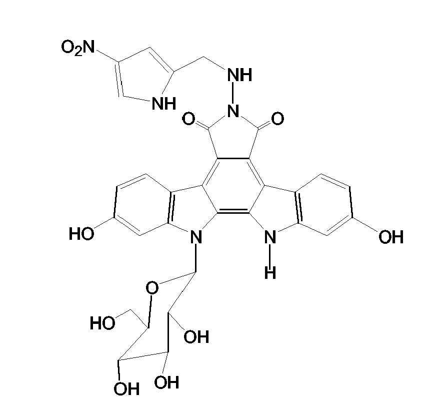

The compound A (29 mg) and 4-nitro-2-pyrrole carboxyaldehyde (20 mg) were dissolved in methanol (3 ml) and then a small amount of acetic acid was added thereto, followed by stirring at 80°C for 21 hours. After the reaction solution was concentrated, the separated solid was washed with ethyl acetate and dried. The resulting solid was suspended in tetrahydrofuran (3 ml) and NaBH₃CN (30 mg) was added thereto, followed by stirring at room temperature. After that, 10% HCl-MeOH (0.3 ml) was dropped thereinto, followed by stirring for 4 hours. An appropriate amount of water was added thereto, followed by extracting with methyl ethyl ketone and washing with a saturated saline solution. The organic phase was concentrated and the residue was filled on a Sephadex LH-20 column chromatography and developed with methanol. The desired fraction was concentrated and dried to give the compound represented by the above formula (8.6 mg).

Rf value: 0.30 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 658 (M)⁺
¹H-NMR (300 MHz, DMSO-d6, δ ppm): 3.48-3.52 (2H, m), 3.73-4.03 (4H, m), 4.16 (2H, d, J = 3.2 Hz), 4.92 (1H, d, J = 4.9 Hz), 5.12 (1H, d, J = 5.1 Hz), 5.34 (1H, d, J = 4.5 Hz), 5.87 (1H, d, J = 3.2 Hz), 5.97 (1H, d, J = 8.3 Hz), 6.15 (1H, bs), 6.59 (1H, s), 6.78-6.84 (2H, m), 6.98 (1H, d, J = 2.4 Hz), 7.17 (1H, d, J = 2.0 Hz), 7.81 (1H, s), 8.75 (1H, d, J = 9.0 Hz), 8.83 (1H, d, J = 8.5 Hz), 9.76 (2H, br), 11.19 (1H, s).

### Examples 11

Synthesis of the compound represented by the following formula

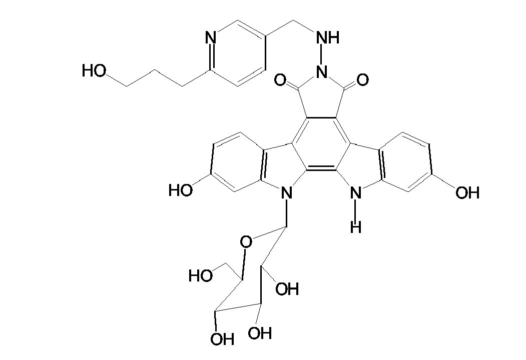

The compound A (160 mg) and 2-(3-benzyloxy-1-propenyl) pyridine-5-carboxyaldehyde (436 mg) were dissolved in methanol (20 ml) and then 20 µl of acetic acid was added thereto, followed by stirring at 80°C for 13 hours. After the reaction solution was concentrated the separated solid was washed with ethyl acetate and dried. The resulting solid (20 mg) was dissolved in methanol-tetrahydrofuran (1:1) (100 ml), 20 mg of 10% palladium-carbon was added and the mixture was stirred in a hydrogen atmosphere for 20 hours. The palladium-carbon was removed by filtration, the reaction solution was concentrated and the residue was purified using Diaion HP-20 column chromatography (methanol) and then using Sephadex LH-20 column chromatography (methanol) to give the compound represented by the above formula (2.4 mg).
FAB (m/z): 684 (M)⁺
¹H-NMR (300 MHz, DMSO-d6, δ ppm): 11.15 (1H, s), 9.76 (2H, br), 8.84 (1H, d, J = 8.6 Hz), 8.75 (1H, d, J = 8.5 Hz), 8.49 (1H, d, J = 2.0 Hz), 7.80 (2H, dd, J = 2.0 Hz, 7.9 Hz), 7.18 (1H, d, J = 7.9 Hz), 7.15 (1H, s), 6.97 (1H, d, J = 1.9 Hz), 6.79 (2H, dt, J = 1.9 Hz, 8.6 Hz), 6.16 (1H, t, J = 4.6 Hz), 5.96 (1H, d, J = 8.1 Hz), 5.86 (1H, bs), 5.35 (1H, bs), 5.12 (1H, bs), 4.90 (1H, bs), 4.40 (1H, bs), 4.23 (2H, d, J = 4.7 Hz), 3.76-4.05 (4H, m), 3.48-3.55 (2H, m), 3.30-3.41 (2H, m), 2.69 (2H, t, J = 7.9 Hz), 1.69-1.70 (2H, m).

### Example 12

Synthesis of the compound represented by the following formula

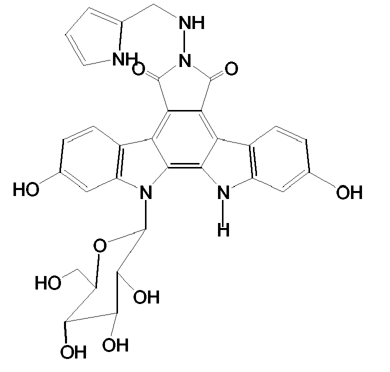

The compound A (30 mg) and 2-pyrrole carboxyaldehyde (50 mg) were dissolved in methanol (3 ml) and then 8 µl of acetic acid was added thereto, followed by stirring at 80°C for 15 hours. After the reaction solution was concentrated, the separated solid was washed with chloroform and dried. The resulting solid was dissolved in tetrahydrofuran (5 ml) and then NaBH₃CN (20 mg) and 10% HCl-MeOH (2 ml) were added thereto, followed by stirring at room temperature for 15 minutes. After the reaction solution was concentrated, the residue was filled on a Sephadex LH-20 column chromatography and developed with methanol. The desired fraction was concentrated and dried to give the compound represented by the above formula (15.0 mg).

Rf value: 0.30 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 613 (M)⁺
¹H-NMR (300 MHz, DMSO-d6, δ ppm): 11.17 (1H, s), 10.73 (1H, s), 9.79 (2H, br), 8.85 (1H, d, J = 8.5 Hz), 8.77 (1H, d, J = 8.6 Hz), 7.16 (1H, s), 6.93 (1H, s), 6.77-6.83 (2H, m), 6.64 (1H, s), 5.80-5.97(5H, m), 5.37 (1H, br), 5.14 (1H, br), 4.92 (1H, br), 4.17 (2H, d, J = 5.1 Hz), 3.75-4.03 (4H, m), 3.49-3.52 (2H, m).

### Example 13

Synthesis of the compound represented by the following formula

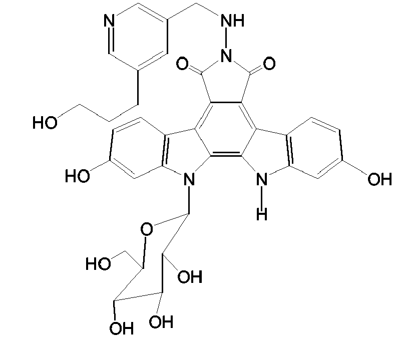

The compound A (200 mg) and 3-(3-hydroxypropyl) pyridine-5-carboxyaldehyde (156 mg) were dissolved in methanol (15 ml) and then 36 µl of acetic acid were added thereto, followed by stirring at 80°C for 13 hours. After the reaction solution was concentrated, the separated solid was washed with ethyl acetate and dried. The resulting solid (20 mg) was dissolved in methanol-tetrahydrofuran (1:1)(100 ml) and then 10 mg of 5% palladium-carbon was added thereto, followed by stirring in a hydrogen atmosphere for 19 hours. After the reaction solution was concentrated and the residue was purified using a Diaion HP-20 column chromatography (methanol) and then using a Sephadex LH-20 column chromatography (methanol) to give the compound represented by the above formula (4.8 mg).

Rf value: 0.20 (Kiesel Gel 60 F₂₅₄ manufactured by Merck; developing solvent was acetonitrile : tetrahydrofuran : toluene : water : acetic acid = 4:2:2:0.5:0.1).
FAB (m/z): 684 (M + H)⁺
¹H-NMR (300 MHz, DMSO-d6, δ ppm): 11.16 (1H, s), 9.77 (2H, br), 8.83 (1H, d, J = 8.5 Hz), 8.75 (1H, d, J = 8.5 Hz), 8.41 (1H, d, J = 2.1 Hz), 8.25 (1H, d, J = 1.5 Hz), 7.79 (1H, s), 7.15 (1H, s), 6.96 (1H, d, J = 2.0 Hz), 6.79 (2H, dt, J = 2.0 Hz, 8.5 Hz), 6.22 (1H, t, J = 4.7 Hz), 5.93 (1H, d, J = 4.9 Hz), 5.87 (1H, br), 5.33 (1H, br), 5.12 (1H, br), 4.89 (1H, br), 4.44 (1H, br), 4.26 (2H, d, J = 4.7 Hz), 3.75-4.04 (4H, m), 3.46-3.52 (2H, m), 2.60 (2H, t, J = 7.8 Hz), 1.65-1.73 (2H, m).

### Example 14

Synthesis of the compound represented by the following formula

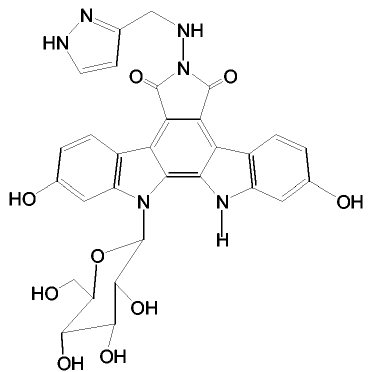

A methanolic solution (2.7 ml) containing the compound A (55 mg) and 3-pyrazole carboxyaldehyde (15 mg) was heated to reflux through the night in the presence of acetic acid (20 µl). The resulting precipitate was collected, washed with methanol, dissolved in methanol (1 ml), THF (1 ml) and DMF (1 ml) with heating; 10% palladium-carbon (30 mg) was added; and the mixture was stirred in a hydrogen stream through the night at room temperature. The reaction mixture was passed through a pad of Celite to remove the palladium-carbon and the filtrate was concentrated. The residue was dissolved in methanol and purified (MeOH) using Sephadex LH-20 (30 ml) to collect the yellow fraction, thereby giving the compound (36.4 mg) represented by the above formula (36.4 mg).
ESI (m/z): 615.2 (M + H)⁺
¹H-NMR (300 MHz, DMSO-d6, δ ppm): 11.29 (1H, s), 9.82 (2H, br), 9.38 (1H, s), 8.89 (1H, d, J = 8.0 Hz), 8.81 (1H, d, J = 8.0 Hz), 7.91 (1H, br), 7.21 (1H, s), 7.02 (1H, s), 6.85 (1H, d, J = 8.0 Hz), 6.80 (1H, d, 8.0 Hz), 5.82-6.08 (2H, m), 5.38 (1H, s), 5.16 (1H, s), 4.99 (1H, br), 4.92 (1H, m), 4.24 (1H, m), 3.76-4.10 (4H, m), 3.43-3.52 (2H, m).

### Example 15

Synthesis of the compound represented by the following formula

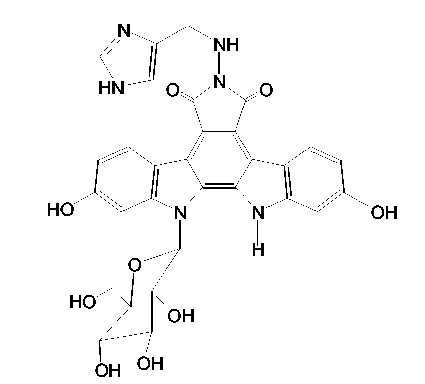

A methanolic solution (2.7 ml) containing the compound A (55 mg) and 4-imidazole carboxyaldehyde (15 mg) was heated to reflux through the night in the presence of acetic acid (20 µl). The resulting precipitate was collected, washed with methanol, dissolved in methanol (1 ml), THF (1 ml) and DMF (1 ml) with heating, 10% palladium-carbon (30 mg) was added and the mixture was stirred in a hydrogen stream through the night at room temperature. The reaction mixture was passed through a pad of Celite to remove the palladium-carbon and the filtrate was concentrated. The residue was dissolved in methanol and purified using a Sephadex LH-20 column chromatography (MeOH : THF = 1:1) to collect the yellow fraction, thereby giving the compound (30.0 mg) represented by the above formula.
APCI (m/z): 615.2 (M + H)⁺
¹H-NMR (300 MHz, DMSO-d6, δ ppm): 11.28(1H, s), 9.86 (2H, br), 9.16 (1H, s), 8.89 (1H, d, J = 8.0 Hz), 8.80 (1H, d, J = 8.0 Hz), 7.88 (1H, s), 7.20 (1H, s), 7.02 (1H, s), 6.86 (2H, s), 6.85 (1H, d, J = 8.0 Hz), 6.80 (1H, d, J = 8.0 Hz), 6.00 (2H, m), 5.44 (1H, brs), 5.20 (1H, br), 5.00 (1H, br), 3.76-4.10 (4H, m), 3.43-3.52 (2H, m).

### Example 16

Synthesis of the compound represented by the following formula

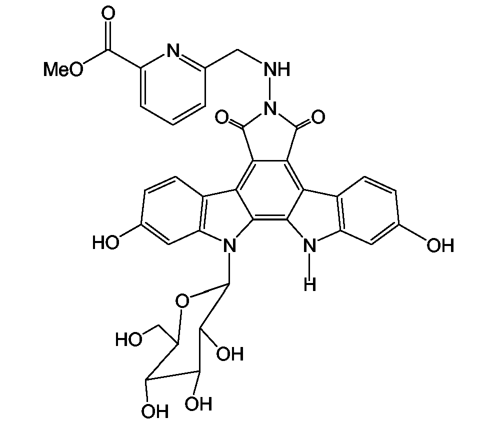

A methanolic solution (3.0 ml) containing the compound A (46 mg) and 2-(6-methoxycarbonylpyridine) carboxyaldehyde (21 mg) was heated to reflux through the night in the presence of acetic acid (15 µl). The resulting precipitate was collected, washed with methanol, dissolved in methanol (2 ml), THF (2 ml) and DMF (1 ml); 10% palladium-carbon (30 mg) was added; and the mixture was stirred in a hydrogen stream through the night at room temperature. The reaction mixture was passed through a pad of Celite to remove the palladium-carbon and the filtrate was concentrated. The residue was dissolved in methanol and purified (MeOH) using a Sephadex LH-20 column chromatography (MeOH : THF = 2:1) to collect the yellow fraction, thereby giving the compound (45 mg) represented by the above formula was obtained.
ESI (m/z): 684.3 (M + H)⁺
¹H-NMR (300 MHz, DMSO-d6, δ ppm): 11.14 (1H, br), 8.82 (1H, d, J = 8.0 Hz), 8.75 (1H, d, J = 8.0 Hz), 8.10 (1H, d, J = 7 Hz), 8.02 (1H, t, J = 7 Hz), 7.90 (1H, d, J = 7 Hz), 7.15 (1H, s), 6.97 (1H, s), 6.82 (1H, d, J = 8.0 Hz), 6.78 (1H, d, J = 8.0 Hz), 6.35 (1H, m), 6.00 (1H, d, J = 8.0 Hz), 4.44 (2H, m), 3.72 (3H, s), 3.76-4.10 (4H, m), 3.43-3.52 (2H, m).

### Example 17

Synthesis of the compound represented by the following formula

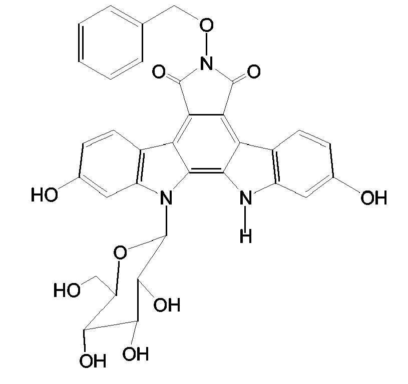

A methanolic solution (4 ml) containing the compound B (48 mg), O-benzylhydroxylamine (35 µl) and triethylamine (120 µl) was heated to reflux for about 5 hours. The reaction mixture was poured over ethyl acetate and washed with 1M HCl. The organic layer was concentrated and the residue was purified by a preparative thin-layer chromatography (methylene chloride : methanol = 4:1) to give the compound (25 mg) represented by the above formula.
APCI (m/z): 626.2 (M + H)⁺
¹H-NMR (300 MHz, DMSO-d6, δ ppm): 11.26 (1H, s), 9.82 (1H, s), 9.80 (1H, s), 8.80 (1H, d, J = 8.0 Hz), 8.74 (1H, d, J = 8.0 Hz), 7.60 (2H, m), 7.42 (3H, m), 7.19 (1H, s), 6.98 (1H, s), 6.85 (1H, d, J = 8.0 Hz), 6.81 (1H, d, J = 8.0 Hz), 5.98 (1H, d, J = 8.0 Hz), 5.88 (1H, br, s), 5.37 (2H, s), 5.16 (1H, d, J = 3.0 Hz), 4.94 (1H, d, J = 3.0 Hz), 4.08 (2H, m), 3.76-4.10 (4H, m), 3.43-3.52 (2H, m).

### Example 18

Synthesis of the compound represented by the following formula

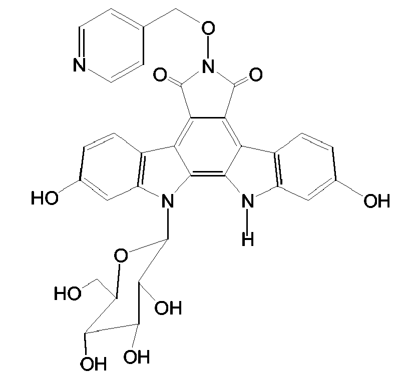

A methanolic solution (3 ml) containing the compound B (50 mg), O-(4-pyridylmethyl)hydroxylamine (25 µl) and triethylamine (10 µl) was heated to reflux through the night. The resulting precipitate was collected and washed with methanol to give the compound (25 mg) represented by the above formula.
ESI (m/z): 627.2 (M + H)⁺
¹H-NMR (300 MHz, DMSO-d6, δ ppm): 11.25 (1H, s), 9.82 (1H, br), 9.80 (1H, br), 8.78 (1H, d, J = 8.0 Hz), 8.72 (1H, d, J = 8.0 Hz), 8.62 (2H, d, J = 4.0 Hz), 7.60 (2H, d, J = 4.0 Hz), 7.19 (1H, s), 6.99 (1H, s), 6.84 (1H, d, J = 8.0 Hz), 6.80 (1H, d, J = 8.0 Hz), 5.3 6 (2H, s), 5.13 (1H, d, J = 4.0 Hz), 4.94 (1H, d, J = 4.0 Hz), 3.76-4.10 (4H, m), 3.43-3.52 (2H, m).

### Example 19

Synthesis of the compound represented by the following formula

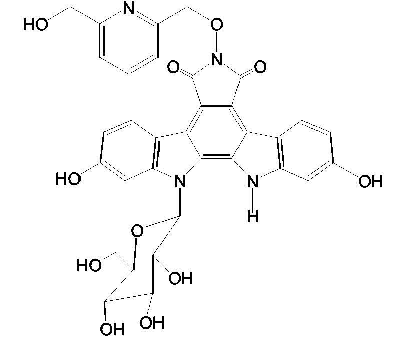

A methanolic solution (3 ml) containing the compound B (57 mg), O-(6-tert-butyldimethylsilyloxymethyl-2-pyridylmethyl) hydroxylamine (35 mg) and triethylamine (10 µl) was heated to reflux through the night. The reaction solution was placed on a Sephadex LH-20 column chromatography (30 ml) and eluted with methanol, and then the fraction of the product (yellow fraction) was collected and concentrated to give 42 mg of the product. This was dissolved in THF (2 ml) and 1M TBAF (0.22 ml) was added thereto, followed by stirring for 2 hours. THF was evaporated *in vacuo;* the residue was placed on a Sephadex LH-20 column chromatography and eluted with methanol; and a fraction of the product (yellow fraction) was collected and concentrated to give the compound (36 mg) represented by the above formula.
ESI (m/z): 657.3 (M + H)⁺
¹H-NMR (300 MHz, DMSO-d6, δ ppm): 11.25 (1H, s), 9.82 (2H, br), 8.78 (1H, d, J = 8.0 Hz), 8.72 (1H, d, J = 8.0 Hz), 7.92 (1H, t, J = 7.0 Hz), 7.66 (1H, d, J = 7.0 Hz), 7.46 (1H, d, J = 7.0 Hz), 7.19 (1H, s), 6.99 (1H, s), 6.84 (1H, d, J = 8.0 Hz), 6.80 (1H, d, J = 8.0 Hz), 5.98 (1H, d, J = 7.0 Hz), 5.89 (1H, br), 5.35 (1H, s), 5.32 (2H, s), 5.13 (1H, d, J = 4.0 Hz), 4.94 (1H, d, J = 4.0 Hz), 4.48 (2H, s), 3.76-4.10 (4H, m), 3.43-3.52 (2H, m).

### Example 20

Synthesis of the compound represented by the following formula

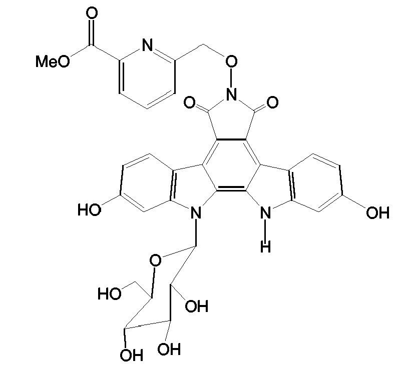

A methanolic solution (4 ml) containing the compound B (57 mg), O-(6-methoxycarbonyl- 2-pyridylmethyl) hydroxylamine (24 mg) and triethylamine (10 µl) was heated to reflux through the night. The reaction solution was placed on a Sephadex LH-20 column chromatography and eluted with methanol, and then the fraction of the product (yellow fraction) was collected and concentrated to give the compound (16 mg) represented by the above formula.
¹H-NMR (300 MHz, DMSO-d6, δ ppm): 11.25 (1H, s), 9.82 (2H, br), 8.76 (1H, d, J = 8.0 Hz), 8.05-8.25 (3H, m), 7.19 (1H, s), 6.98 (1H, s), 6.85 (1H, d, J = 8.0 Hz), 6.81 (1H, d, J = 8.0 Hz), 6.00 (1H, d, J = 8.0 Hz), 5.89 (1H, br), 5.42 (2H, s), 5.34 (1H, s), 5.13 (1H, d, J = 4.0 Hz), 4.92 (1H, d, J = 4.0 Hz), 4.39 (2H, s), 3.76 (3H, s), 3.76-4.10 (4H, m), 3.43-3.52 (2H, m).

### Example 21

Synthesis of the compound represented by the following formula

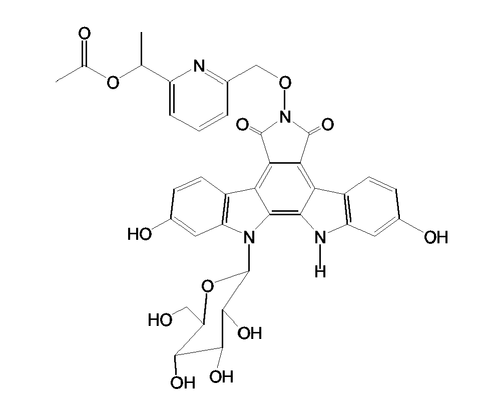

A methanolic solution (3 ml) containing the compound B (50 mg), O-(6-(2-acetoxyethyl)-2-pyridylmethyl) hydroxylamine (30 mg) and triethylamine (10 µl) was heated to reflux through the night. The reaction solution was placed on a Sephadex LH-20 column chromatography and eluted with methanol, and then the fraction of the product (yellow fraction) was collected and concentrated to give the compound (37 mg) represented by the above formula.
ESI (m/z): 713.3 (M+H)⁺

### Example 22

Synthesis of the compound represented by the following formula

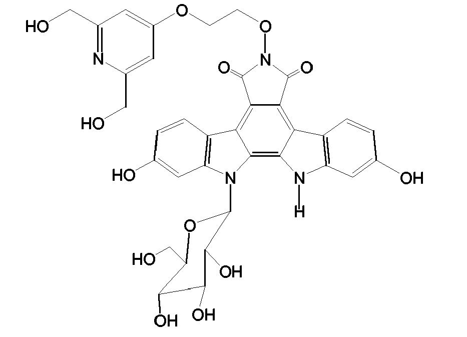

A methanolic solution (3 ml) containing the compound B (40 mg), an excessive amount of O-(2,6-bis(tert- butyldimethylsilyloxymethyl)-4-pyridylmethyl) hydroxylamine and triethylamine (10 µl) was heated to reflux through the night. The reaction solution was placed on a Sephadex LH-20 column chromatography and eluted with methanol and the fraction of the product (yellow fraction) was collected and concentrated to give 36 mg of the product. This was dissolved in 2 ml of THF and 1M TBAF (0.30 ml) was added thereto, followed by stirring for 1 hour. THF was evaporated *in vacuo* and the residue was placed on a Sephadex LH-20 column chromatography and eluted with methanol, and then a fraction of the product (yellow fraction) was collected and concentrated to give the compound (23 mg) represented by the above formula.
ESI (m/z): 717.3 (M+H)⁺
¹H-NMR (300 MHz, DMSO-d6, δ ppm): 11.25 (1H, s), 9.82 (1H, s), 9.80 (1H, s), 8.76 (1H, d, J = 8.0 Hz), 8.70 (1H, d, J = 8.0 Hz), 7.19 (1H, s), 6.99 (1H, s), 6.87 (2H, s), 6.85 (1H, d, J = 8.0 Hz), 6.79 (1H, d, J = 8.0 Hz), 5.98 (1H, d, J = 8.0 Hz), 5.89 (1H, br), 5.33 (3H, m), 5.13 (1H, d, J = 4.0 Hz), 4.60 (1H, s), 4.44 (2H, s), 4.42 (2H, s), 3.76-4.18 (4H, m), 3.43-3.52 (2H, m).

### Example 23

Synthesis of the compound represented by the following formula

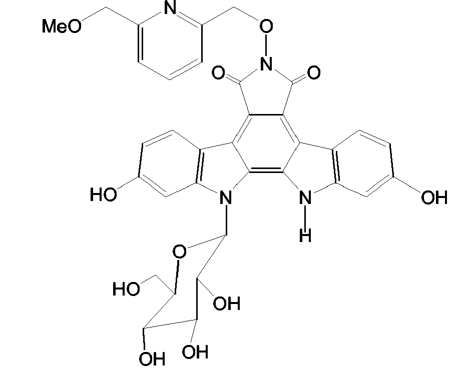

A methanolic solution (4 ml) containing the compound B (50 mg), O-(6-methoxyethyl-2-pyridylmethyl) hydroxylamine (60 mg) and triethylamine (10 µl) was heated to reflux through the night. The reaction solution was placed on a Sephadex LH-20 column chromatography and eluted with methanol, and the fraction of the product (yellow fraction) was collected and concentrated to give 38 mg of the product.
ESI (m/z): 671.3 (M + H)⁺
¹H-NMR (300 MHz, DMSO-d6, δ ppm): 11.24 (1H, s), 9.81 (2H, br), 8.77 (1H, d, J = 8.0 Hz), 8.69 (1H, d, J = 8.0 Hz), 7.93 (1H, t, J = 7.0 Hz), 7.71 (1H, d, J = 7.0 Hz), 7.39 (1H, d, J = 7.0 Hz), 7.19 (1H, s), 7.13 (1H, d, J = 3.0 Hz), 6.98 (1H, s), 6.85 (1H, d, J = 8.0 Hz), 6.81 (1H, d, J = 8.0 Hz), 5.98 (1H, d, J = 8.0 Hz), 5.89 (1H, br), 5.34 (1H, s), 5.33 (2H, s), 5.13 (1H, d, J = 4.0 Hz), 4.94 (1H, d, J = 4.0 Hz), 4.39 (2H, s), 3.76-4.10 (4H, m), 3.43-3.52 (2H, m), 3.23 (3H, s).

### Example 24

Synthesis of the compound represented by the following formula

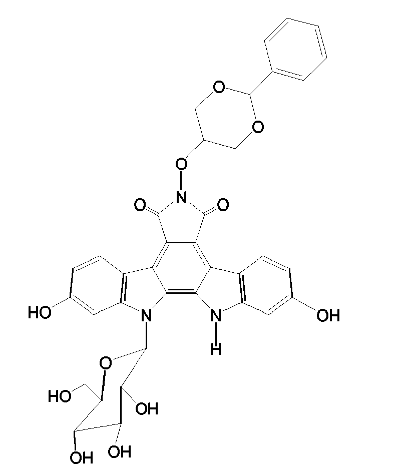

A methanolic solution (4 ml) containing the compound B (86 mg), O-(2-phenyl-1,3-dioxan-5-yl) hydroxylamine (97 mg) and triethylamine (20 µl) was heated to reflux for 12 hours. The reaction solution was placed on a Sephadex LH-20 column chromatography and eluted with methanol-THF (2:1), and then the fraction of the product (yellow fraction) was collected and concentrated to give the product represented by the above formula (51 mg).
APCI (m/z): 698.3 (M + H)⁺
¹H-NMR (300 MHz, DMSO-d6, δ ppm): 11.28 (1H, s), 9.84 (2H, br), 8.82 (1H, d, J = 8.0 Hz), 8.75 (1H, d, J = 8.0 Hz), 7.41 (2H, m), 7.35 (3H, m), 7.20 (1H, s), 7.00 (1H, s), 6.86-6.81 (2H, m), 6.01 (1H, d, J = 8.0 Hz), 5.90 (1H, br, s), 5.63 (1H, s), 5.35 (1H, br), 5.13 (1H, br), 4.94 (1H, br), 4.53 (2H, m), 3.76-4.10 (5H, m), 3.43-3.52 (2H, m).

### Example 25

Synthesis of the compound represented by the following formula

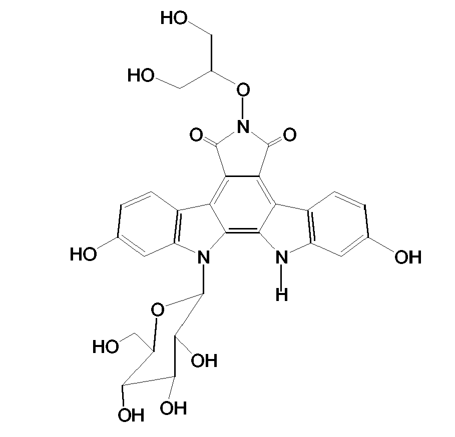

The compound (51 mg) obtained in Example 24 was dissolved in methanol-THF (1:2) (3 ml) and stirred at 35°C for 12 hours in a hydrogen stream (45 psi) in the presence of palladium black (145 mg). After the palladium black was filtered off, the solvent was evaporated *in vacuo;* the residue was placed on a Sephadex LH-20 column chromatography and eluted with methanol; and the fraction of the product (yellow fraction) was collected and concentrated *in vacuo* to give the product represented by the above formula (12 mg).
APCI (m/z): 610.2 (M + H)⁺
¹H-NMR (300 MHz, DMSO-d6, δ ppm): 11.25 (1H, s), 9.82 (2H, br), 8.80 (1H, d, J = 8.0 Hz), 8.73 (1H, d, J = 8.0 Hz), 7.19 (1H, s), 6.99 (1H, s), 6.85 (1H, d, J = 8.0 Hz), 6.81 (1H, d, J = 8.0 Hz), 5.98 (1H, d, J = 8.0 Hz), 5.89 (1H, br), 5.35 (1H, br), 5.13 (1H, s), 4.94 (1H, d, J = 4.0 Hz), 4.74 (1H, br), 4.26 (1H, t, J = 4.0 Hz), 3.76-4.10 (8H, m), 3.43-3.52 (2H, m).

### Example 26

Synthesis of the compound represented by the following formula

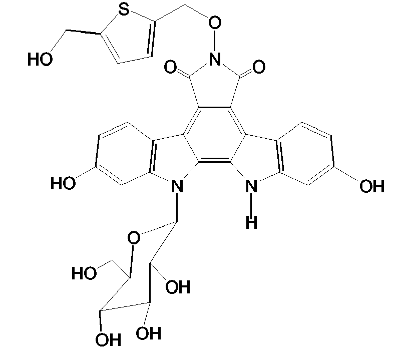

A methanolic solution (4 ml) containing the compound B (100 mg), O-(5-hydroxymethyl-2-thiophenylmethyl) hydroxylamine (159 mg) and triethylamine (30 µl) was heated to reflux through the night. The reaction solution was placed on a Sephadex LH-20 column chromatography and eluted with methanol-THF (1:1), and then the fraction of the product (yellow fraction) was collected and concentrated to give 206 mg of solid. The resulting solid (53 mg) was dissolved in THF (2 ml) and 1M TBAF (0.30 ml) was added thereto, followed by stirring for 2 hours. THF was evaporated *in vacuo;* the residue was placed on a Sephadex LH-20 column chromatography and eluted with methanol-THF (1:1); and a fraction of the product (yellow fraction) was collected and concentrated *in vacuo* to give the compound (25 mg) represented by the above formula.
ESI (m/z): 660.4 (M - H)⁻
¹H-NMR (300 MHz, DMSO-d6, δ ppm): 11.25 (1H, s), 9.81 (2H, br), 8.80 (1H, d, J = 8.0 Hz), 8.72 (1H, d, J = 8.0 Hz), 7.19 (1H, s), 7.13 (1H, d, J = 3.0 Hz), 6.98 (1H, s), 6.85 (1H, d, J = 8.0 Hz), 6.81 (1H, d, J = 8.0 Hz), 5.98 (1H, d, J = 8.0 Hz), 5.88 (1H, br), 5.37 (1H, br), 5.13 (1H, s), 4.94 (1H, d, J = 4.0 Hz), 4.61 (2H, s), 3.76-4.10 (4H, m), 3.43-3.52 (2H, m).

### Example 27

Synthesis of the compound represented by the following formula

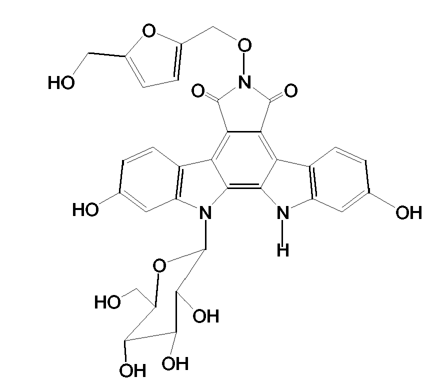

A methanolic solution (4 ml) containing the compound B (91 mg), O-(5-hydroxymethyl-2-furanylmethyl) hydroxylamine (135 mg) and triethylamine (20 µl) was heated to reflux through the night. The reaction solution was placed on a Sephadex LH-20 column chromatography and eluted with methanol-THF (1:1), and then the fraction of the product (yellow fraction) was collected and concentrated to give 80 mg of solid. This was dissolved in THF (2.4 ml) and 1M TBAF (0.31 ml) was added thereto, followed by stirring for 2 hours. THF was evaporated *in vacuo;* the residue was placed on a Sephadex LH-20 column chromatography and eluted with methanol; and a fraction of the product (yellow fraction) was collected and concentrated *in vacuo* to give the compound (64 mg) represented by the above formula.
ESI (m/z): 644.5(M - H)⁻
¹H-NMR (300 MHz, DMSO-d6, δ ppm): 11.24 (1H, s), 9.82 (2H, br), 8.79 (1H, d, J = 8.0 Hz), 8.72 (1H, d, J = 8.0 Hz), 7.19 (1H, s), 6.99 (1H, s), 6.84 (1H, d, J = 8.0 Hz), 6.81 (1H, d, J = 8.0 Hz), 6.60 (1H, d, J = 2.5 Hz), 6.27 (1H, d, J = 2.5 Hz), 5.98 (1H, d, J = 8.0 Hz), 5.89 (1H, br), 5.34 (1H, s), 5.29 (1H, t, J = 4.0 Hz), 5.18 (2H, s), 5.13 (1H, s), 4.94 (1H, d, J = 4.0 Hz), 4.38 (2H, d, J = 4.0 Hz), 3.76-4.10 (4H, m), 3.43-3.52 (2H, m).

### Example 28

Synthesis of the compound represented by the following formula

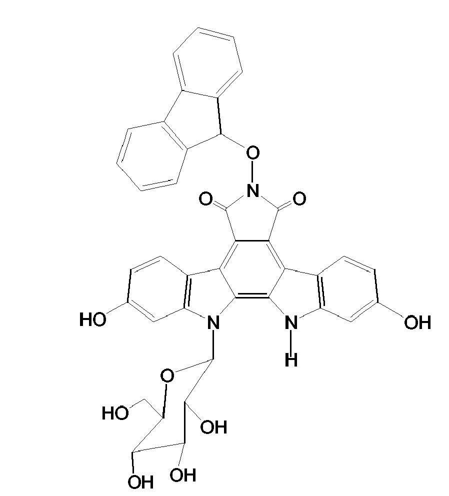

A methanolic solution (6 ml) containing the compound B (137 mg), O-(9H-fluoren-9-yl) hydroxylamine (156 mg) and triethylamine (40 µl) was heated to reflux through the night. The reaction solution was placed on a Sephadex LH-20 column chromatography and eluted with methanol, and then the fraction of the product (yellow fraction) was collected and concentrated to give the compound (36 mg) represented by the above formula.
APCI (m/z): 700.3(M + H)⁺
¹H-NMR (300 MHz, DMSO-d6, δ ppm): 11.29 (1H, s), 9.82 (2H, br), 8.84 (1H, d, J = 8.5 Hz), 8.78 (1H, d, J = 8.5 Hz), 7.98 (2H, m), 7.85 (2H, d, J = 7.5 Hz), 7.49 (2H, t, J = 7.5 Hz), 7.40 (2H, t, J = 7.5 Hz), 7.17 (1H, s), 7.01 (1H, s), 6.86 (1H, d, J = 8.0 Hz), 6.81 (1H, d, J = 8.0 Hz), 6.39 (1H, s), 6.02 (1H, m), 5.88 (1H, br), 5.91 (1H, m), 5.36 (1H, s), 5.13 (1H, m), 4.94 (1H, m), 3.76-4.10 (4H, m), 3.43-3.52 (2H, m).

### Example 29

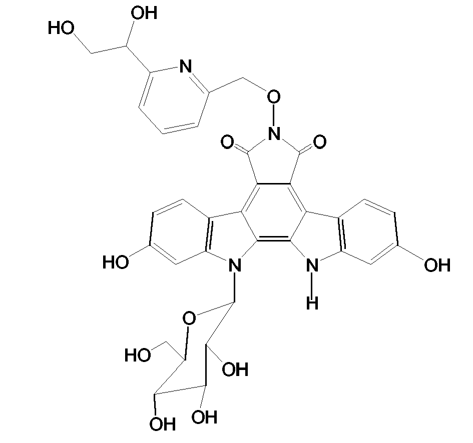

A methanolic solution (5 ml) containing the compound B (131 mg), O-(6-(1,2-diacetoxyethyl)-2-pyridylmethyl) hydroxylamine (203 mg) and triethylamine (40 µl) was heated to reflux through the night. The reaction solution was placed on a Sephadex LH-20 column chromatography and eluted with methanol, and then yellow fraction was collected to give 124 mg of solid. The resulting solid (45 mg) was dissolved in methanol (4.5 ml) ; 1M NaOH (260 µl) was added under ice cooling, followed by stirring for 30 minutes. After 1M HCl (130 µl) was added thereto, the mixture was placed on a Sephadex LH-20 column chromatography (30 ml) and eluted with methanol, and then the fraction of the product (yellow fraction) was collected and concentrated *in vacuo* to give the compound (25 mg) represented by the above formula.
ESI (m/z): 687.3 (M + H)⁺
¹H-NMR (300 MHz, DMSO-d6, δ ppm): 11.24 (1H, s), 9.82 (2H, br), 8.76 (1H, d, J = 8.7 Hz), 8.70 (1H, d, J = 8.7 Hz), 8.04 (1H, t, J = 7.6 Hz), 7.79 (1H, d, J = 7.6 Hz), 7.60 (1H, d, J = 7.6 Hz), 7.19 (1H, s), 6.99 (1H, s), 6.86-6.80 (2H, m), 5.98 (1H, d, J = 8.2 Hz), 5.40 (2H, s), 4.62 (1H, m), 4.40-3.40 (10H, m).

### Example 30

Synthesis of the compound represented by the following formula

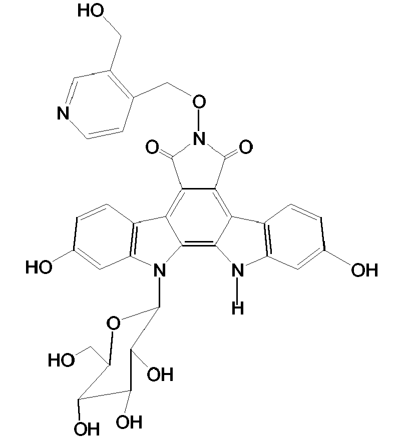

A methanolic solution (4 ml) containing the compound B (97.1 mg), O-(3-tert-butyldimethylsiloxymethyl-4-pyridylmethyl) hydroxylamine (54.3 mg) and triethylamine (30 µl) was heated to reflux for 3 days. The reaction solution was concentrated *in vacuo;* the resulting residue was dissolved in a mixed solvent of THF (4 ml) and methanol (3 ml); 1M TBAF (1 ml) was added thereto; the reaction solution was stirred at room temperature for 1.5 hours; 1M TBAF (1 ml) was added thereto again; and the reaction solution was stirred at room temperature for 1 hour. 1M TBAF (1 ml) was further added thereto, and the reaction solution was stirred at room temperature for 30 minutes and heated to reflux for 30 minutes. The reaction solution was concentrated *in vacuo* and the resulting residue was subjected to a Sephadex LH-20 column chromatography to give the compound (11 mg) represented by the above formula as orange solid.
ESI (m/z): 657.1 (M + H)⁺, 655.2 (M-H)⁻
¹H-NMR (400 MHz, DMSO-d6, δ ppm): 11.23 (1H, br), 9.82 (2H, br), 8.77 (1H, d, J = 8.4 Hz), 8.70 (1H, d, J = 8.4 Hz), 8.63 (1H, s), 8.53 (1H, d, J = 5.2 Hz), 7.60 (1H, d, J = 5.2 Hz), 7.18 (1H, s), 6.98 (1H, s), 6.80-6.84 (2H, m), 5.93-5.99 (2H, m), 5.30-5.41 (5H, m), 5.16 (1H, br), 4.96 (1H, br), 4.82 (1H, br), 3.50-4.08 (6H, m).

### Example 31

Synthesis of the compound represented by the following formula

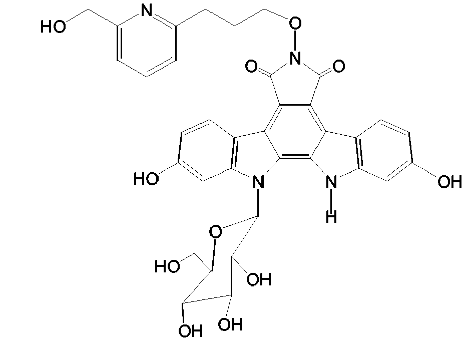

A methanolic solution (5 ml) containing the compound B (130 mg), O-(2-(6-tert-butyldiphenylsiloxymethyl)pyridylmethyl) hydroxylamine (447 mg) and triethylamine (40 µl) was heated to reflux through the night. The reaction solution was placed on a Sephadex LH-20 column chromatography (30 ml) and eluted with methanol, and then a fraction of the product (yellow fraction) was collected and concentrated to give 70.2 mg of solid. This was dissolved in THF (2.8 ml) and 1M TBAF (0.35 ml) was added thereto, followed by stirring for 2 hours. THF was evaporated *in vacuo;* the residue was placed on a Sephadex LH-20 column chromatography and eluted with methanol; and a fraction of the product (yellow fraction) was collected and concentrated *in vacuo* to give the compound (50.5 mg) represented by the above formula.
ESI (m/z): 685.3 (M + H)⁺
¹H-NMR (300 MHz, DMSO-d6, δ ppm): 11.25 (1H, s), 9.82 (2H, br), 8.80 (1H, d, J = 8.6 Hz), 8.73 (1H, d, J = 8.6 Hz), 7.71 (1H, t, J = 7.8 Hz), 7.28 (1H, d, J = 7.8 Hz), 7.20 (1H, d, J = 7.8 Hz), 7.19 (1H, s), 6.99 (1H, s), 6.77-6.86 (2H, m), 5.98 (1H, d, J = 8.2 Hz), 5.89 (1H, br), 5.3 5 (2H, m), 5.13 (1H, d, J = 4.2 Hz), 4.93 (1H, d, J = 4.2 Hz), 4.51 (2H, d, J = 5.6 Hz), 4.28 (2H, t, J = 6.4 Hz), 3.76-4.10 (5H, m), 3.43-3.52 (2H, m), 2.96 (2H, t, J = 7.8 Hz), 2.13 (2H, m).

### Example 32

Synthesis of the compound represented by the following formula

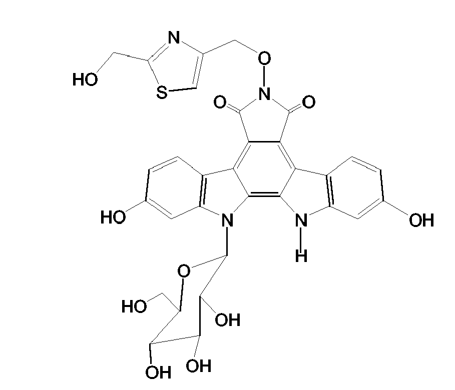

A methanolic solution (4 ml) containing the compound B (100.2 mg), O-(4-(2-tert-butyldiphenylsiloxymethyl)thiazolylmethyl) hydroxylamine (87.1 mg) and triethylamine (30 µl) was heated to reflux for 2 hours. To this was added triethylamine (100 µl) and the mixture was further heated to reflux for 14 hours. The reaction solution was concentrated *in vacuo;* the resulting residue was dissolved in methanol (3 ml); 1M TBAF (0.8 ml) was added thereto; and the reaction solution was heated to reflux for 2 days. The reaction solution was concentrated *in vacuo* and the resulting residue was subjected to a Sephadex LH-20 column chromatography using methanol as an eluting solvent to give the compound (12 mg) represented by the above formula as orange solid.
ESI (m/z): 663.2 (M + H)⁺, 661.2 (M - H)⁻
¹H-NMR (400 MHz, DMSO-d6, δ ppm): 8.76 (1H, d, J = 8.5 Hz), 8.69 (1H, d, J = 8.5 Hz), 7.83 (1H, s), 7.18 (1H, s), 6.98 (1H, s), 6.78-6.83 (2H, m), 5.83-6.00 (2H, m), 5.40 (1H, br), 5.27 (2H, s), 5.20 (1H, br), 4.94 (1H, br), 4.68 (2H, s), 3.22-4.03 (7H, m).

### Example 33

Synthesis of the compound represented by the following formula

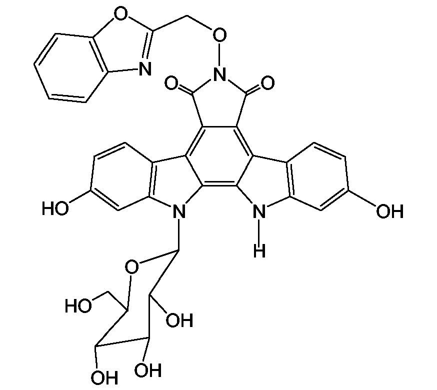

A methanolic solution (3 ml) containing the compound B (89.5 mg), O-(2-benzoxazolylmethyl) hydroxylamine (59 mg) and triethylamine (80 µl) was heated to reflux for 18 hours. The reaction solution was concentrated *in vacuo* and the resulting residue was subjected to a Sephadex LH-20 column chromatography using methanol as an eluting solvent to give the compound (10 mg) represented by the above formula as orange solid.
ESI (m/z): 667.2 (M + H)⁺, 665.3 (M - H)⁻
¹H-NMR (400 MHz, DMSO-d6, δ ppm): 11.25 (1H, br), 9.85 (2H, br), 8.66 (1H, d, J = 8.8 Hz), 8.58 (1H, d, J = 8.8 Hz), 7.81 (1H, d, J = 8.4 Hz), 7.73 (1H, d, J = 8.4 Hz), 7.45-7.49 (1H, m, 7.36-7.40 (1H, m), 7.18 (1H, s), 6.99 (1H, s), 6.76-6.81 (2H, m), 5.93-6.00 (2H, m), 5.53 (2H, m), 5.39 (1H, br), 5.17 (1H, br), 4.95-4.97 (1H, m), 3.45-4.03 (6H, m).

### Example 34

Synthesis of the compound represented by the following formula

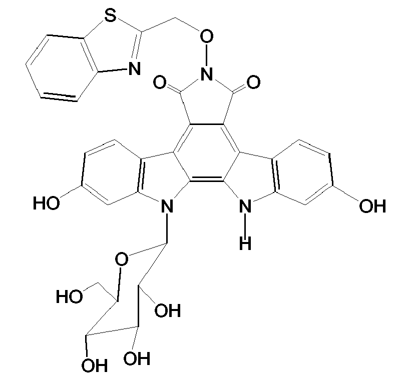

A DMF solution (1 ml) containing the compound B (96.1 mg), O-(2-benzthiazolylmethyl) hydroxylamine (71.5 mg) and triethylamine (200 µl) was heated with stirring for 18 hours. The reaction solution was concentrated *in vacuo,* and the resulting residue was subjected to a Sephadex LH-20 column chromatography using methanol as an eluting solvent to give the compound (19.1 mg) represented by the above formula as orange solid.
ESI (m/z): 683.2 (M + H)⁺, 681.2 (M - H)⁻
¹H-NMR (400 MHz, DMSO-d6, δ ppm): 8.74 (1H, d, J = 8.0 Hz), 8.67 (1H, d, J = 9.2 Hz), 8.19 (1H, d, J = 8.0 Hz), 8.00 (1H, d, J = 8.0 Hz), 7.46-7.56 (2H, m), 7.18 (1H, s), 6.98 (1H, s), 6.78-6.83 (2H, m), 5.97-5.99 (2H, m), 5.73 (2H, m), 5.34 (1H, br), 5.16 (1H, br), 4.96 (1H, br), 3.50-4.03 (6H, m).

### Example 35

Synthesis of the compound represented by the following formula

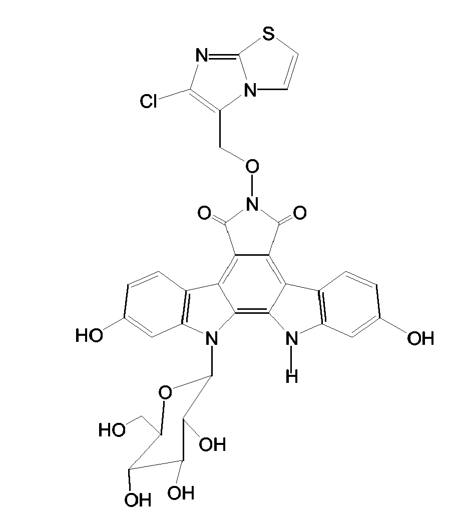

A DMF solution (1 ml) containing the compound B (102.3 mg), O-(6-chloro-5-imidazo[2.1-b][1.3]thiazolylmethyl) hydroxylamine (42.3 mg) and triethylamine (100 µl) was heated with stirring at 80°C for 18 hours. The reaction solution was concentrated *in vacuo,* and the resulting residue was subjected to a Sephadex LH-20 column chromatography using methanol as an eluting solvent to give the compound (12.1 mg) represented by the above formula as orange solid.
ESI (m/z): 706.2 (M + H)⁺, 704.3 (M - H)⁻
¹H-NMR (400 MHz, DMSO-d6, δ ppm): 11.22 (1H, br), 9.84 (2H, br), 8.71 (1H, d, J = 8.4 Hz), 8.65 (1H, d, J = 7.7 Hz), 8.26 (1H, d, J = 5.0 Hz), 7.55 (1H, d, J = 5.0 Hz), 7.18 (1H, s), 6.99 (1H, s), 6.79-6.88 (2H, m), 5.92-6.07 (2H, m), 5.51 (2H, s), 5.40 (1H, br), 5.18 (1H, br), 4.98 (1H, br), 3.22-4.03 (6H, m).

### Example 36

Synthesis of the compound represented by the following formula

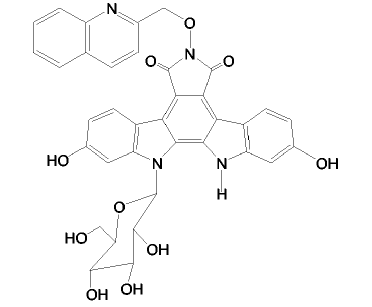

A DMF solution (1 ml) containing the compound B (101.2 mg), O-(2-quinolylmethyl) hydroxylamine (40.7 mg) and triethylamine (100 µl) was heated with stirring at 80°C for 18 hours. The reaction solution was concentrated *in vacuo,* and the resulting residue was subjected to a Sephadex LH-20 column chromatography using methanol as an eluting solvent to give the compound (11.5 mg) represented by the above formula as orange solid.
ESI (m/z): 677.2 (M + H)⁺, 675.3 (M - H)⁻
¹H-NMR (400 MHz, DMSO-d6, δ ppm): 11.20 (1H, br), 8.85 (1H, dd, J = 1.8 Hz, 4.4 Hz), 8.73 (1H, d, J = 8.7 Hz), 8.66 (1H, d, J = 8.4 Hz), 8.38 (1H, dd, J = 1.5 Hz, 8.1 Hz), 8.09 (1H, d, J = 7.0 Hz), 8.01 (1H, d, J = 7.0 Hz), 7.66 (1H, d, J = 8.1 Hz), 7.52 (1H, dd, J = 4.0 Hz, 8.1 Hz), 7.17 (1H, d, J = 1.8 Hz), 6.97 (1H, d, J = 2.2 Hz), 6.77-6.82 (2H, m), 5.82-5.96 (4H, m), 5.38 (1H, br), 5.15 (1H, br), 4.95 (1H, br), 3.04-4.10 (6H, m).

### Example 37

Synthesis of the compound represented by the following formula

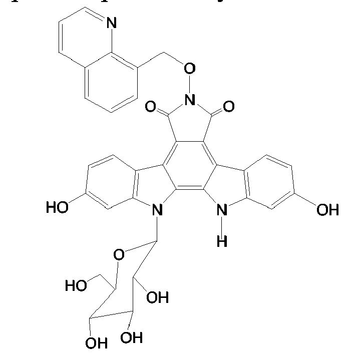

A DMF solution (1 ml) containing the compound B (85.9 mg), O-(8-quinolylmethyl) hydroxylamine (109.8 mg) and triethylamine (100 µl) was heated with stirring at 80°C for 3 days. The reaction solution was *concentrated in vacuo,* and the resulting residue was subjected to a Sephadex LH-20 column chromatography using methanol as an eluting solvent and then purified again by means of HPLC (eluting solvent: acetonitrile-water containing 0.1% TFA) to give the compound (2.5 mg) represented by the above formula as orange solid.
ESI (m/z): 677.2 (M + H)⁺, 675.3 (M - H)⁻
¹H-NMR (400 MHz, DMSO-d6, δ ppm): 11.22 (1H, br), 9.81 (2H, br), 8.74 (1H, d, J = 8.6 Hz), 8.66 (1H, d, J = 8.6 Hz), 8.50 (1H, d, J = 8.0 Hz), 7.93-8.09 (3H, m), 7.71-7.74 (1H, m), 7.57-7.70 (1H, m), 7.15 (1H, s), 6.95 (1H, s), 6.77-6.81 (2H, m), 5.95 (1H, d, J = 8.8 Hz), 5.52 (2H, s), 3.22-4.03 (6H, m).

### Example 38

Synthesis of the compound represented by the following formula

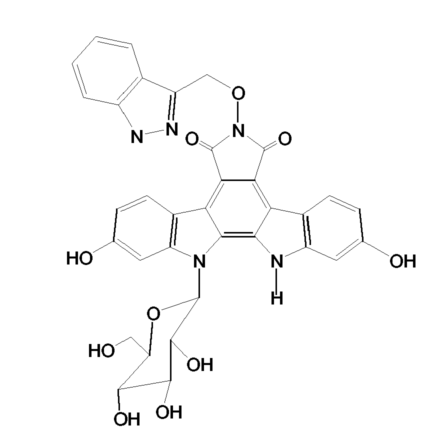

A DMF solution (1 ml) containing the compound B (37.8 mg), O-(3-indazolylmethyl) hydroxylamine (211.5 mg) and triethylamine (100 µl) was heated with stirring at 80°C for 14 hours. The reaction solution was concentrated *in vacuo,* and the resulting residue was developed on a Sephadex LH-20 column chromatography and eluted with methanol. A fraction of the product (yellow fraction) was collected and concentrated *in vacuo* to give the compound (12.3 mg) represented by the above formula.
ESI (m/z): 666.3 (M + H)⁺, 664.3 (M - H)⁻
¹H-NMR (400 MHz, DMSO-d6, δ ppm): 11.21 (1H, br), 8.78 (1H, d, J = 8.8 Hz), 8.71 (1H, d, J = 8.2 Hz), 8.15 (1H, d, J = 8.4 Hz), 7.54 (1H, d, J = 8.4 Hz), 7.38-7.42 (1H, m), 7.15-7.26 (2H, m), 6.98 (1H, s), 6.80-6.85 (2H, m), 5.97-6.12 (2H, m), 5.59 (2H, s), 5.35 (1H, br), 5.20 (1H, br), 4.90 (1H, br), 3.15-4.04 (6H, m).

### Example 39

Synthesis of the compound represented by the following formula

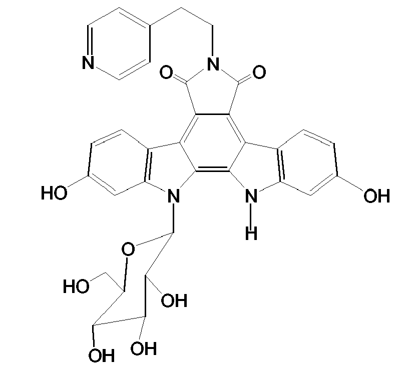

An N-methylpyrrolidone solution (1 ml) containing the compound B (81.5 mg),4-(2-aminoethyl)pyridine (100 mg) and triethylamine (100 µl) was heated with stirring at 80°C for 14 hours. The reaction solution was concentrated *in vacuo,* and the resulting residue was developed on a Sephadex LH-20 column chromatography and eluted with methanol. A fraction of the product (yellow fraction) was collected and concentrated *in vacuo* to give the compound (20.8 mg) represented by the above formula.
ESI (m/z): 625.3 (M + H)⁺, 623.3 (M - H)⁻
¹H-NMR (400 MHz, DMSO-d6, δ ppm): 11.16 (1H, br), 8.80 (1H, d, J = 8.8 Hz), 8.72 (1H, d, J = 8.4 Hz), 8.41 (2H, d, J = 5.5 Hz), 7.29 (2H, d, J = 5.5 Hz), 7.15 (1H, s), 6.97 (1H, s), 6.77-6.81 (2H, m), 5.94-5.96 (2H, m), 5.42 (1H, br), 5.20 (1H, br), 4.96 (1H, br), 3.15-4.04(10H, m).

### Industrial Applicability

The compound according to the present invention exhibits remarkable cell-growth suppressive effect against cancer cells. Therefore, it is useful as an anti-tumor agent in the field of medicines.

## Claims

1. A compound of the following formula or a pharmaceutically acceptable salt thereof: 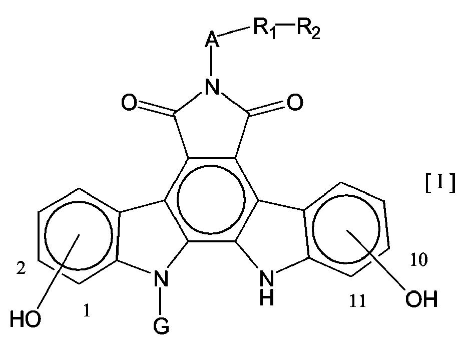 wherein:
A represents O, NH, or CH₂;
R₁ represents a single bond, a lower alkyl group, a lower alkenyl group, a lower alkynyl group, or a formula of Y₁- W, wherein Y₁ represents a lower alkyl group, a lower alkenyl group or dioxanyl; W represents a single bond or an oxygen atom; said lower alkyl group, lower alkenyl group, or lower alkynyl group may be substituted with one or more substituent(s), which is/are the same or different, selected from the group consisting of <substituent group β>;
R₂ represents a phenyl group, a naphthyl group, or a five- or six-membered aromatic or aliphatic heterocyclic ring having at least one atom selected from N, S, or O, selected from the group consisting of <substituent group α>, wherein said phenyl group, naphthyl group, aromatic or aliphatic heterocyclic ring may be substituted with one or more substituent(s), which is/are the same or different, selected from the group consisting of <substituent group β> and/or a lower alkyl group substituted with one or more substituent(s), which is/are the same or different, selected from the group of <substituent group β>; when A represents an oxygen atom, R₂ may represent hydrogen atom; with a proviso that A is NH and R₁ is CH₂, R₂ is not any of a substituted phenyl group, a hydroxymethyl-substituted naphthyl group, an unsubstituted pyridyl group, a hydroxymethyl-substituted pyridyl group, an unsubstituted thienyl group, a hydroxymethyl-substituted 2-thienyl group, a mono(hydroxymethyl)-substituted 3-thienyl group, an unsubstituted furyl group and a hydroxymethyl-substituted furyl group;
G represents a hexose group or a pentose group;
<substituent group α> represents the following: 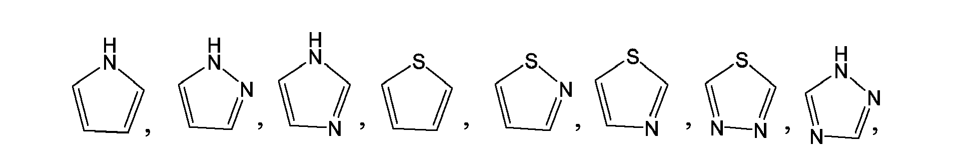 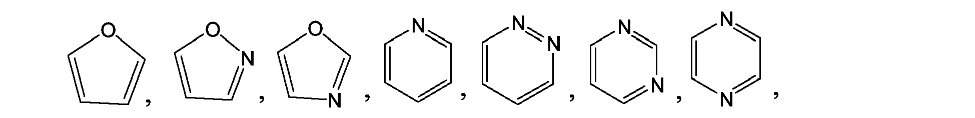 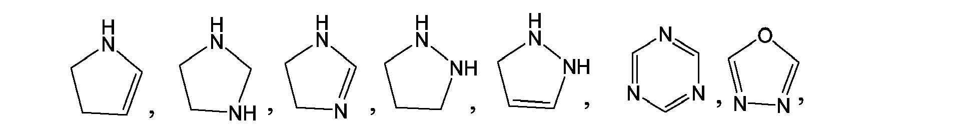 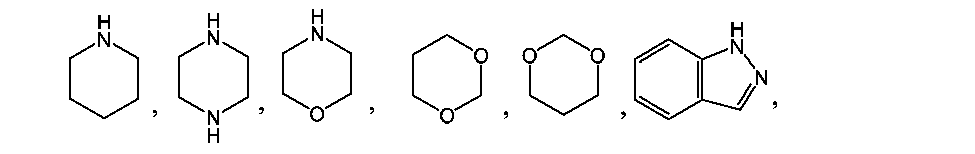 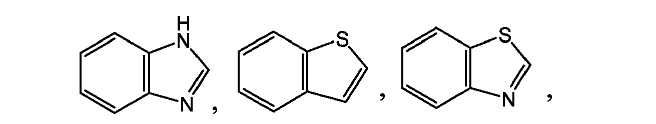 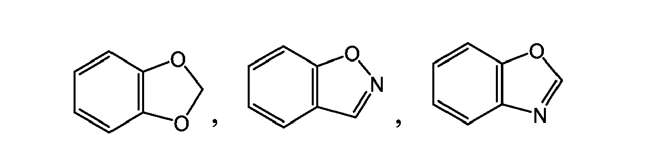 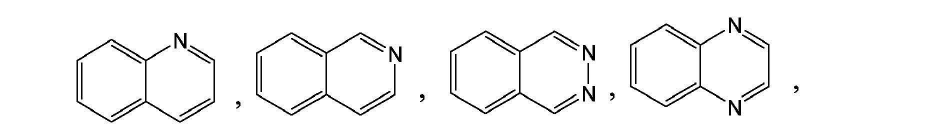 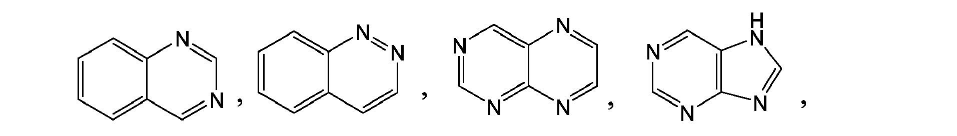 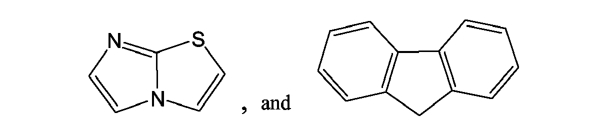
and; <substituent group β> represents the following:
a hydroxyl group, a cyano group, a halogen atom, a nitro group, a carboxyl group, a carbamoyl group, a formyl group, a lower alkanoyl group, a lower alkanoyloxy group, a lower alkoxy group, a hydroxyl lower alkoxy group, a lower alkoxy carbonyl group, a lower alkyl carbamoyl group, a di-lower alkyl carbamoyl group, a carbamoyloxy group, a lower alkyl carbamoyloxy group, a di-lower alkyl carbamoyloxy group, an amino group, a lower alkyl amino group, a di-lower alkyl amino group, a tri-lower alkyl ammonio group, a lower alkanoyl amino group, an aroyl amino group, a lower alkanoyl amidino group, a hydroxyl imino group, a lower alkoxy imino group, a lower alkyl thio group, a lower alkyl sulfinyl group, a lower alkyl sulfonyl group, a lower alkyl sulfonylamino group and a sulfamoyl group.

2. The compound according to claim 1 or a pharmaceutically acceptable salt thereof, wherein G represents β-glucopyranosyl group; the positions of substitution of the hydroxyl groups on the indolopyrrolocarbazole ring are the 2- and 10-positions; R₁ represents a lower alkyl group; and R₂ represents a five- or six-membered aromatic or aliphatic heterocyclic ring having at least one atom selected from N, S, or O, selected from the group consisting of the <substituent group α>.

3. The compound according to claim 2 or a pharmaceutically acceptable salt thereof, wherein the <substituent group α> represents the following: 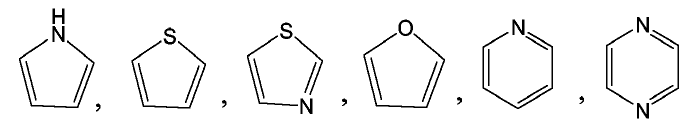 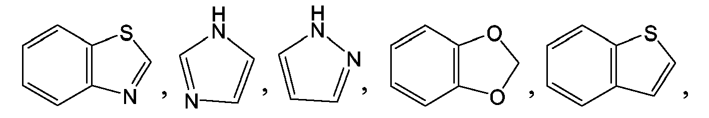 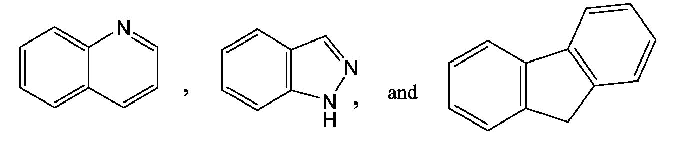 and the <substituent group β> represents the following:
a hydroxyl group, a nitro group, a lower alkanoyl group, a lower alkanoyloxy group, a lower alkoxy group, and a lower alkoxy carbonyl group.

4. The compound according to claim 3 or a pharmaceutically acceptable salt thereof, wherein A represents O; the <substituent group α> represents the following: 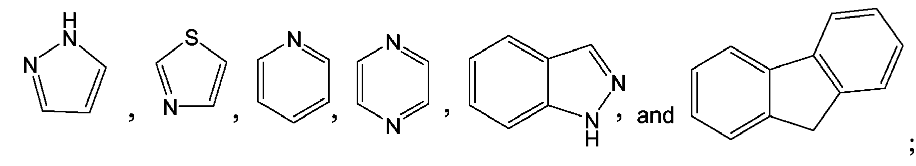 and the <substituent group β> represents the following:
a hydroxyl group, a lower alkanoyl group, a lower alkanoyloxy group, and a lower alkoxy carbonyl group.

5. The compound according to claim 3 or a pharmaceutically acceptable salt thereof, wherein A represents NH or CH₂; the <substituent group α> represents the following: 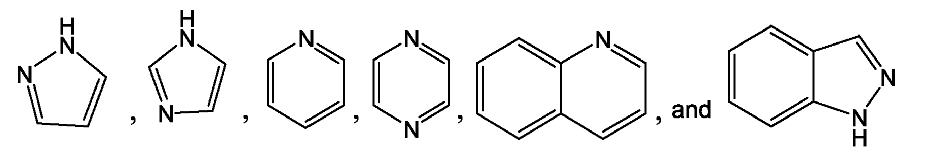 ; and the <substituent group β> represents the following:
a hydroxyl group, a lower alkanoyl group, and a lower alkyl carbonyloxy group.

6. A compound according to claim 1 which is:
6-N-(2,5-dihydroxymethyl-3-thienylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-pyrazinylmethylamino-12,13-dihydro-2,10-dihydroxy-12-β-D-glucopyranosyl-5H-indolo-[2,3-a]pyrrolo[3,4-c]carbazole-5,7(6H)-dione;
6-N-(4-quinolinylmethylamino)-12,13 -dihydro-2,10-dihydroxy-12-β-D-glucopyranosyl-5H-indolo[2,3-a]pyrrolo[3,4-c]carbazole-5,7(6H)-dione;
6-N-(4-nitro-2-1H-pyrrolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(2-1H-pyrrolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(3-1H-pyrazolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(4-1H-imidazolylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(2-methoxycarbonyl-6-pyridylmethyl)amino-12,13-dihydro-2,10-dihydroxy-12-β-D-glucopyranosyl-5H-indolo[2,3-a]pyrrolo[3,4-c]carbazole-5,7(6H)-dione;
6-N-(4-pyridylmethoxy)-12,13-dihydro-2,10-dihydroxy-12-β-D-glucopyranosyl-5H-indolo-[2,3-a]pyrrolo[3,4-c]carbazole-5,7(6H)-dione;
6-N-(6-hydroxymethyl-2-pyridylmethoxy)-12,13-dihydro-2,10-dihydroxy-12-β-D-glucopyranosyl-5H-indolo[2,3-a]pyrrolo[3,4-c]carbazole-5,7(6H)-dione;
6-N-(3-hydroxymethyl-4-pyridylmethoxy)-12,13-dihydro-2,10-dihydroxy-12-β-D-glucopyranosyl-5H-indolo[2,3-a]pyrrolo[3,4-c]carbazole-5,7(6H)-dione; or
6-N-(2-(4-pyridyl)ethyl)-12,13-dihydro-2,10-dihydroxy-12-β-D-glucopyranosyl-5H-indolo-[2,3-a]pyrrolo[3,4-c]carbazole-5,7(6H)-dione,
or a pharmaceutically acceptable salt thereof.

7. A pharmaceutical composition comprising the compound according to any one of claims 1 to 6 as an active ingredient, together with a pharmaceutically acceptable carrier or diluent.

8. An antitumor agent comprising the compound according to any one of claims 1 to 6 as an active ingredient, together with a pharmaceutically acceptable carrier or diluent.
